(19) Europäisches Patentamt
European Patent Office
Office européen des brevets

(11) **EP 2 892 441 B1**

(12) **EUROPÄISCHE PATENTSCHRIFT**

(45) Veröffentlichungstag und Bekanntmachung des Hinweises auf die Patenterteilung:
**02.11.2016 Patentblatt 2016/44**

(51) Int Cl.:
*A61B 17/16* *(2006.01)*          *A61B 17/32* *(2006.01)*
*B23B 31/00* *(2006.01)*          *B23B 31/107* *(2006.01)*

(21) Anmeldenummer: **13752646.3**

(22) Anmeldetag: **26.08.2013**

(86) Internationale Anmeldenummer:
**PCT/EP2013/067639**

(87) Internationale Veröffentlichungsnummer:
**WO 2014/037238 (13.03.2014 Gazette 2014/11)**

(54) **CHIRURGISCHES, DREHMOMENT ÜBERTRAGENDES INSTRUMENT EINSCHLIESSLICH ZUGEHÖRIGEM WERKZEUG**

SURGICAL, TORQUE-TRANSMITTING INSTRUMENT INCLUDING ASSOCIATED TOOL

INSTRUMENT CHIRURGICAL TRANSMETTANT UN COUPLE ET COMPRENANT UN OUTIL CORRESPONDANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.09.2012 DE 102012108264**

(43) Veröffentlichungstag der Anmeldung:
**15.07.2015 Patentblatt 2015/29**

(73) Patentinhaber: **Aesculap AG**
**78532 Tuttlingen (DE)**

(72) Erfinder:
• **BARTH, Jürgen**
**78588 Denkingen (DE)**

• **KRAFT, Florian**
**78579 Neuhausen ob Eck (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(56) Entgegenhaltungen:
**DE-A1- 4 103 663     DE-B3- 10 311 455**
**US-A- 5 934 846      US-A1- 2011 098 688**

## Beschreibung

Die vorliegende Erfindung betrifft ein chirurgisches Instrument für das Bereitstellen eines Drehmoment sowie ein angetriebenes, drehbar im Instrumentenhandstück bzw. daran angeschlossenen Handstückschaft gelagertes Werkzeug, auf das ein möglichst hohes Drehmoment übertragbar ist.

## Hintergrund der Erfindung

In der modernen (minimal invasiven) Chirurgie werden Instrumente beispielsweise zur spanabhebenden bzw. materialabtragenden Bearbeitung von Knochen, Knorpeln etc., beispielsweise bei arthroskopischen Eingriffen, in der Wirbelsäulenchirurgie und dergleichen orthopädischen Behandlungen verwendet, welche ein ergonomisch geformtes Handstück und ein ggf. austauschbares Werkzeug (z.B. ein Fräser, Drehmesser, Polierkopf, etc.) umfassen, das im Handstück an dessen distalem Ende drehbar sowie angetrieben gelagert ist. Als Werkzeugantrieb ist je nach Verwendungszweck und beabsichtigter Werkzeugdrehzahl ein hydraulischer, pneumatischer oder elektromotorischer Antrieb vorgesehen, der über einen Drehmoment-Übertragungszug (beispielsweise ein Getriebe und/oder eine Anzahl ggf. miteinander gekoppelter Wellen) innerhalb des Handstücks mit dem Werkzeug wirkverbunden ist. Die Antriebe können dabei im Handstück integriert sein oder sie sind als externe Antriebseinheiten ausgebildet, welche über Energieversorgungsleitungen oder Drehmoment-Übertragungsstränge (z.B. biegeelastische Wellen) mit dem Handstück gekoppelt sind, wobei in diesem Fall das Handstück im Wesentlichen lediglich zur Unterbringung des Getriebes oder Drehmoment-Übertragungszugs dient.

An den Handstücken sind an deren körperzugewandten (distalen) Enden in der Regel rohrförmige Handstückschäfte angeschlossen/montiert, die je nach Einsatzzweck unterschiedliche Schaftlängen und - formen haben, um an unterschiedliche Stellen innerhalb eines Patientenkörpers vorzudringen. So existieren beispielsweise gerade, bogenförmig gekrümmte oder vorzugsweise im Montagebereich mit dem Handstück gekröpfte (abgewinkelte) Handstückschäfte, in denen jedoch immer eine Drehmoment-Übertragungswelle oder - schaft (nachfolgend Torsionswelle) gelagert ist. Diese Welle/Schaft muss starr (torsionssteif) genug sein, um das erforderliche Drehmoment an ein daran distal eingesetztes / ausgebildetes Werkzeug übertragen zu können (d.h. die Welle muss eine ausreichende Torsionssteifigkeit besitzen), jedoch biegsam genug sein, d.h. auch eine gewisse Biegeflexibilität aufweisen, um den Krümmungen eines (nicht geraden) Handstückschaft-Verlaufs auch bei einer drehenden Bewegung folgen zu können.

Zur Verbindung des Werkzeugs mit der im Handstückschaft gelagerten Torsionswelle ist eine Wellenkupplung zur lösbaren Aufnahme eines Werkzeugschafts vorgesehen. Eine Schwierigkeit besteht hierbei jedoch darin, eine derartige Wellenkupplung für das ggf. austauschbare Werkzeug innerhalb des kleindurchmessrigen Handstückschafts so zu gestalten, dass auch bei derart kleinen Handstückschaft-Durchmessern und hohen Drehzahlen eine sichere und langlebige Funktion des chirurgischen Instruments insbesondere auch im Fall von langen Handstückschäften gewährleistet ist. Außerdem sollte der Handstückschaft ebenfalls austauschbar am Handstück aufgenommen sein, um unterschiedliche Schaftlängen und - formen mit einem einzigen Handstück realisieren zu können. Entscheidend hierfür ist die weitere lösbare Drehmomentverbindung zwischen dem im Handstück untergebrachten Getriebe / Drehmomentübertragungszug und der im Schaft gelagerten Torsionswelle, welche zum Einen einfach und sicher schließbar sein muss und zum Anderen ausreichend hohe Drehmomente übertragen sollte. Schließlich sollte die Bedienbarkeit des Instruments einschließlich des Wechsels eines Werkzeugs und/oder eines Handstückschafts einfach und sicher sein.

## Stand der Technik

Beispielsweise aus der DE 103 11 455 A1 ist ein chirurgisches Instrument dieser Gattung und insbesondere ein Handstück eines solchen chirurgischen Instruments bekannt.

Das bekannte Handstück besteht in diesem besonderen Fall aus einem hülsenförmigen Griffabschnitt (könnte natürlich auch eine andere Griffform haben), an dessen proximalem (körperabgewandtem) Ende ein Leitungspaket zur Energieversorgung (Druckluft, elektrischer Strom oder Hydraulikdruck) anschließbar ist und an dessen distalem (körperzugewandtem) Ende ein Handstückschaft mittels einer Überwurfmutter (ggf. auswechselbar) aufgeschraubt ist. Der Handstückschaft hat eine äußere sowie innere Schafthülle, die auch zur Gleit-Drehführung einer darin eingeführten Torsionswelle dient. Die innere Schafthülle ist in Axialrichtung in mehrere Teilabschnitte unterteilt, zwischen denen jeweils ein Kugellager in die äußere Schafthülle eingesetzt ist, welche die Torsionswelle gegen die äußere Schafthülle abstützen. Am distalen Ende der Torsionswelle ist ein Werkzeug, vorzugsweise ein Fräskopf fixiert/ausgebildet.

Wie aus dieser Druckschrift zu entnehmen ist, wird das Werkzeug grundsätzlich aus einem Eingriffs- bzw. Schneidkopf und der Torsionswelle gebildet, die einstückig miteinander verbunden sind. Die Kopplung zwischen Werkzeug und Getriebe/Drehmomentübertragungszug innerhalb des Griffabschnitts erfolgt demnach ausschließlich im Bereich der Überwurfmutter. Dies bedeutet, dass das Werkzeug eine speziell an diesen einen Handstückschaft längenangepasste Einzelanfertigung ist, die nicht für andere Handstückschäfte unterschiedlicher Längen verwendbar ist. Es ist daher offensichtlich, dass ein solches Konstruktionsprinzip teuer in der Herstellung wie auch in der Bereitstellung ist, da für jeden Handstückschaft passendes Werkzeug vorhanden/vorgehalten sein muss.

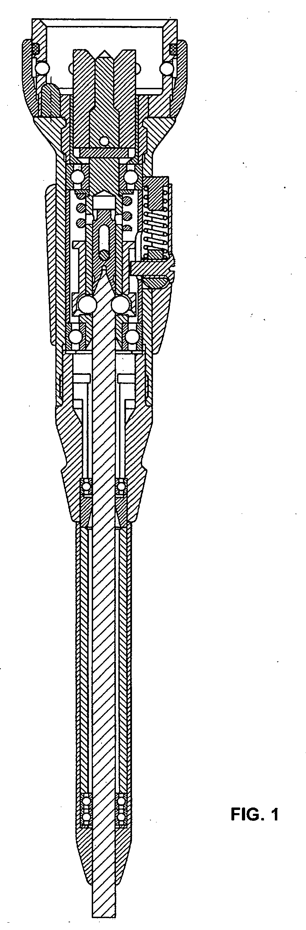

Aus der anliegenden Fig. 1 ist der Längsschnitt eines solchen bekannten chirurgischen Instruments mit bereits eingesetztem Werkzeug schematisch dargestellt.

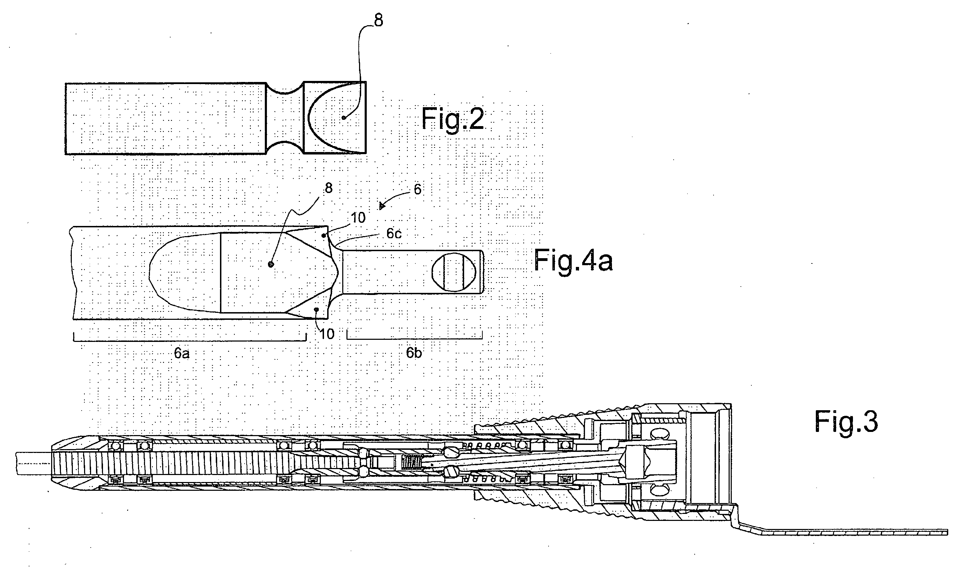

Demnach hat das bekannte Werkzeug den Werkzeugschaft, der aus dem distalen Ende des Instrumenten-/Handstückschafts drehbar herausragt und an dessen distalem Ende ein (nicht weiter dargestellter) Schneidkopf ausgebildet ist. Am in der Fig. 2 in Vergrößerung dargestellten proximalen Werkzeugschaftende ist der bekannte Werkzeugschaft keilförmig angeschärft, wodurch zwei voneinander abgewandte Schrägflächen zur Drehmomenteinleitung ausgebildet werden (entspricht einem sogenannten Zweiflach). Am distalen Endbereich dieser Keilform ist der Werkzeugschaft mit einer umlaufenden Nut ausgeformt, welche als Axialsicherung dient, wie dies nachfolgend noch beschrieben wird.

Das bekannte Handstück ist entsprechend der vorstehenden Werkzeugkonstruktion an seinem distalen Ende mit einer axial verschiebbaren Überwurfhülse versehen, über die der Instrumenten-/Handstückschaft am Handstück drehfest ankoppelbar ist. Innerhalb des Handstücks im Bereich der Überwurfhülse ist ein drehbar gelagertes Aufnahmerohr vorgesehen, das an seinem proximalen Endabschnitt wiederum in einer Drehwelle steckt und dort mittels eines Querstifts drehfest gesichert ist.

Am distalen Ende des Aufnahmerohrs sind zumindest zwei diametral sich gegenüberliegende Bohrungen ausgeformt, in welche Klemmkugeln beweglich eingesetzt sind. Um die Außenseite des Aufnahmerohrs ist eine Schließ- oder Klemmhülse axialverschiebbar gelagert, die in einer ersten Axialposition die Klemmkugeln für eine Bewegung radial nach Außen freigibt und in einer zweiten Axialposition die Klemmkugeln radial nach innen drückt. Zur manuellen Betätigung der Klemmhülse ist ein weiterer Schieber vorgesehen, der an der Außenseite des Handstücks gelagert und über einen Mitnahmestift mit der Klemmhülse verbunden ist. Dabei sei noch erwähnt, dass der Schieber in Richtung zweiter Axialposition der Klemmhülse federvorgespannt ist.

Wie ferner aus der Fig. 1 zu ersehen ist, befindet sich innerhalb des Aufnahmerohrs ein axial relativverschiebbarer Drehmoment-Übertragungsbolzen mit einer distal angeordneten, axial sich erstreckenden, keilförmigen Kerbe, die mit der Keilform des Werkzeugschafts in Drehmoment-Übertragungseingriff bringbar ist. Der Bolzen ist dabei mittels einer Feder in Richtung distal vorgespannt und durch einen Querstift drehfest im Aufnahmerohr gesichert.

Gemäß diesem konstruktiven Aufbau muss das bekannte Werkzeug mit seinem Werkzeugschaft voraus an der distalen Spitze des Handstückschafts in diesen eingeführt und in Richtung zum Aufnahmerohr axial darin verschoben werden, bis der proximale Werkzeugschaftteil (Zweifach) an den Klemmkugeln anliegt. Jetzt wird die Klemmhülse über den Schieber in deren Freigabeposition axial verschoben, sodass der Werkzeugschaftteil die Klemmkugeln radial nach Außen verdrängen und so weiter in das Aufnahmerohr einfahren kann, bis er in der Kerbe des Drehmoment-Übertragungsbolzens zu liegen kommt. Wird jetzt der Schieber wieder freigegeben. Dabei bewegt sich die Klemmhülse eigenständig (angetrieben durch die Vorspannfeder) in deren Klemmposition zurück, in welcher die Klemmkugeln radial nach Innen in die Umfangsnut am Werkzeugschaft gedrückt werden und damit den Werkzeugschaft axial verriegeln. Auf diese Weise kann nunmehr ein Drehmoment von der Drehwelle über den Querstift, das Aufnahmerohr, den weiteren Querstift und den Drehmoment-Übertragungsbolzen (Drehmoment-Übertragungszug) auf den Werkzeugschaft gebracht werden.

Die vorstehend beschriebene, bekannte Konstruktion hat jedoch einige verbesserungswürdige Besonderheiten:

Die beschriebenen Querstiftverbindungen bewirken eine lokale Materialschwächung am Drehmoment-Übertragungsbolzen und an dem Aufnahmerohr wie auch an der Drehwelle innerhalb des Handstücks. Außerdem sind die Querstifte relativ dünn und brechen daher schnell. Insgesamt ist daher das übertragbare Drehmoment eingeschränkt.

Die Keilform des Werkzeugschafts ist ebenfalls nicht gut geeignet, hohe Drehmomente zu übertragen, da der axial wirkende Kraftanteil ein Lösen der Werkzeugseitigen Keilform von der Kerbe des Drehmoment-Übertragungsbolzens bewirkt. Darüber hinaus ist mit einem Aufspreizen des Aufnahmerohrs im Bereich der Kerbe zu rechnen.

Schließlich ist die gesamte Kupplungsmechanik zur Verbindung des Werkzeugschafts mit dem Getriebe/Drehmomentübertragungszugs innerhalb des InstrumentenHandstücks in den Bereich der Überwurfhülse verlagert, wo ggf. noch ausreichend radialer Platz zur Aufnahme der Kupplungselemente besteht. Dadurch müssen aber die Werkzeugschäfte die gesamte Länge der Instrumenten-/Handstückschäfte überbrücken. D.h. für jeden Handstückschaft sind spezielle Werkzeuge erforderlich. Die US 2011 / 098 688 A1 offenbart ein Instrument nach dem Oberbegriff des Anspruchs 1.

**Kurzbeschreibung der Erfindung**

Angesichts dieses Stands der Technik ist es die Aufgabe der vorliegenden Erfindung, ein chirurgisches, Drehmoment übertragendes Instrument oder Instrumentensystem vorzugsweise bestehend aus einem Instrumentenhandstück und zumindest einem (oder mehreren) Werkzeug(en) bereit zu stellen, mit welchem jeweils eine höhere Funktionalität erreichbar ist. Vorzugsweise soll das Instrument insgesamt hohe Drehmomente übertragen können und weiter vorzugsweise einfach und sicher bedienbar sein. Vorteilhaft wäre es auch, wenn durch die Verwendbarkeit universeller Werkzeuge für unterschiedliche (auswechselbare) Handstückschäfte die Herstellungs- und Bereithaltungskosten für das System/Instrument gesenkt werden könnten.

Die vorstehende Aufgabe sowie die weiteren vorteilhaften Ziele der Erfindung werden durch ein gattungsgemäßes einem System/Instrument aus Handstück und vorzugsweise austauschbarem Handstückschaft mit den Merkmalen des Anspruchs 1 gelöst. Vorteilhafte Ausgestaltungen und/oder Weiterbildungen der Erfindung, welche ggf. auch unabhängig voneinander zu beanspruchen sind, sind Gegenstand der Unteransprüche.

Ein Aspekt der vorliegenden Erfindung sieht demzufolge die Bereitstellung eines chirurgischen, Drehmoment übertragenden Instruments vor mit einem Handstück, an dem ein Handstückschaft angeflanscht oder anflanschbar ist (umfasst auch die einstückige Ausformung des Handstückschafts mit dem Handstück), in welchem eine Werkzeugaufnahme für ein wahlweises Aufnehmen eines drehbar im Handstückschaft gelagerten oder lagerbaren chirurgischen Werkzeugs vorgesehen ist insbesondere zur axialen Sicherung des Werkzeugs in der Werkzeugaufnahme sowie zur Übertragung eines Drehmoments auf das Werkzeug. Die Werkzeugaufnahme hat erfindungsgemäß u.a. die folgenden Bauteile:

- eine hülsenförmige Mitnehmerwelle mit einem proximalen Einsteckbereich für eine ein Drehmoment einleitende Torsionswelle sowie distalen Drehmomentübertragungs- und Axialverriegelungsbereichen für das Werkzeug und

- eine die Mitnehmerwelle verschiebbar sowie vorzugsweise drehbar umgebende Schließhülse mit einem distalen Sperrabschnitt, der für ein axiales Verriegeln und Freigeben des Werkzeugs auf den Axialverriegelungsbereich der Mitnehmerwelle einwirkt und einen proximalen Arretierabschnitt, der für ein Drehmomente sowie Axialkräfte übertragendes Arretieren und Lösen der Werkzeugaufnahme an und von der Torsionswelle auf den Einsteckbereich der Mitnehmerwelle einwirkt.

Dadurch, dass Drehmomente unmittelbar von der Mitnehmerwelle auf das Werkzeug (beispielsweise durch Formschluss zwischen beiden Bauteilen übertragen wird, reduziert sich in diesem Bereich die Zahl an in den Drehmomentübertragungsfluss eingebundenen Bauteile/Elemente, sodass der radial zu Verfügung stehende Bauraum (innerhalb des Handstückschafts) maximal zur Drehmomentübertragung ausnutzbar ist. Des Weiteren erfolgt die Drehmpmentübertragung zwischen Torsionswelle und Mitnehmerwelle am Außenumfang der Torsionswelle (durch Einwirkung der Schließhülse) unter Ausnutzung eines großen Hebelarms in radialer Richtung. Somit können auch an dieser Stelle vergleichsweise hohe Drehmomente übertragen werden.

Vorzugsweise ist ein oder mehrere in Umfangsrichtung beabstandete Mitnahmeelemente vorgesehen, die im Einsteckbereich zwischen die Mitnehmerwelle und die Torsionswelle (ausschließlich in deren Umfangs-Randbereich) formschlüssig eingefügt sind und durch die Schließhülse in einer axialen Verriegelungsposition radial nach Außen gesichert oder sicherbar sind. Dadurch wird die Torsionswelle nur minimal materiell geschwächt und kann so große Drehmomente übertragen, ohne radial aufgeweitet werden zu müssen.

Gemäß einem weiteren, ggf. unabhängigen Aspekt der vorliegenden Erfindung kann es vorgesehen sein, dass die Mitnahmeelemente vorzugsweise in Form von Kugeln, oder stirnseitig abgerundeten Walzen, Zylindern oder Tonnen in Aufnahmetaschen (ausschließlich) am Außenumfang der Torsionswelle sowie in radialen Durchbrüchen oder Öffnungen im Einsteckbereich der Schließhülse sitzen, um von der Torsionswelle zumindest Drehmomente und vorzugsweise auch Axialkräfte (unmittelbar) auf die Mitnehmerwelle zu übertragen. Hierdurch lässt sich eine vergleichweise großflächige Anlage zwischen Mitnahmeelementen und der Torsionswelle sowie der Mitnehmerwelle erreichen, um ein Abscheren der Mitnahmeelemente bei hohen Drehmomenten zu vermeiden.

Zur Erleichterung und Vereinfachung der Montage der erfindungsgemäßen Werkzeugaufnahme kann es bevorzugt vorgesehen sein, die Schließhülse in ihrem proximalen Arretierabschnitt zumindest mit eine rund vorzugsweise mit einer Anzahl von umfangsseitigen Befüllöffnungen auszubilden, für das Einsetzen der Mitnahmeelemente, wenn sich die Schließhülse in ihrer einen (kombinierten) axialen Freigabe- sowie rotatorischen Befüllposition befindet. D.h. es sind in der Schließhülse Befüllöffnungen vorgesehen die bei der korrekten Axial- und Drehposition bezüglich der Mitnehmerwelle mit deren Durchbrüchen überlappen und so das radiale Einsetzen der Mitnahmeelemente in die Durchbrüche sowie die radial innen liegenden Taschen der Torsionswelle erlauben. Nach Abschluss des Befüllvorgangs braucht die Schließhülse nur noch (in deren ausschließliche axiale Freigabeposition) zurückgedreht und dann in deren axiale Werkzeug - Verriegelungsposition verschoben zu werden.

Gemäß einem weiteren ggf. unabhängig zu beanspruchenden Aspekt der Erfindung kann es vorgesehen sein, dass der distale Drehmoment-Übertragungsbereich (24a) durch einen axialen Längsschlitz in die zumindest in diesem Abschnitt hülsenförmige Mitnehmerwelle gebildet ist, wodurch zwei axial sich erstreckende, einen Werkzeugaufnahmespalt definierende Vorsprünge oder Zungen entstehen, die für eine Formschlussverbindung mit einem in den Spalt eingesetzten Werkzeug vorgesehen sind. Dabei sei erwähnt, dass die beiden Zungen vorzugsweise von einem Radiallager (Kugellager) zumindest mit Innenring umgeben sind, um ein radiales Aufspreizen bei einer Drehmomentübertragung zu vermeiden.

Ein weiterer, ggf. unabhängig beanspruchbarer Aspekt der Erfindung kann vorsehen, dass im proximalen Anschluss an den Drehmoment-Übertragungsbereich der vorstehend bereits genannte Axialverriegelungsbereich angeordnet ist, unter anderem bestehend aus zumindest einer, vorzugsweise einer Anzahl winkelbeabstandeter Radial-Durchgangsbohrungen, in die jeweils ein Verriegelungselement vorzugsweise in Form einer Kugel eingesetzt sind, welche durch die Schließhülse in ihrer axialen Verriegelungsposition radial nach innen (gegen den Werkzeugschaft) gedrückt werden. Vorzugsweise hat die Schließhülse hierfür an ihrem distalen Endabschnitt eine innere radiale Aufweitung zum radialen Freigeben der Kugeln in ihrer axialen Freigabeposition und zum Befüllen der Radial-Durchgangsbohrungen mit den Verriegelungskugeln in ihrer axialen Freigabe- sowie rotatorischen Befüllposition (gemäß vorstehender Definition).

Ein weiterer, ggf. unabhängiger Aspekt der vorliegenden Erfindung kann eine in der Mitnehmerwelle ausgeformte Aufnahmekammer proximal zum Verriegelungsbereich für ein Nachfahrelement vorsehen, das einen axialen, vorzugsweise bolzenförmigen Eingriffsabschnitt hat, der durch eine axial wirkende Nachfahrfeder in Richtung radial zwischen die vorzugsweise kugelförmigen Verriegelungselemente gedrängt wird.

Vorteilhaft ist es hierbei auch, wenn das Nachfahrelement so ausgebildet ist, um bei nicht eingesetztem Werkzeug die Verriegelungselemente radial nach Außen zu drücken und dabei die Schließhülse in ihrer axialen Freigabeposition zu halten und durch Einsetzten eines Werkzeugs axial gegen die Nachfahrfeder verschoben zu werden, um ein radiales Einrücken der Verriegelungselemente für ein Axialverriegeln des Werkzeugs sowie ein axiales Verschieben der Schließhülse in ihre Verriegelungsposition zu erlauben, in welcher die Verriegelungselemente radial einwärts gegen das Werkzeug gedrückt bleiben.

Durch diese Maßnahmen entsteht eine Art halbautomatische Werkzeugaufnahme, welche zunächst in ihrer Freigabeposition verharrt, in welcher ein Werkzeug einsetzbar ist, wodurch (durch den Einsetzvorgang) automatisch ein Verriegeln des Werkzeugs für eine gesicherte Drehmomentübertragung ausgelöst wird (erfolgt). Durch manuelles axiales Verschieben der Schließhülse in ihre Freigabeposition wird das Werkzeug für dessen Entnahme wieder freigegeben.

Schließlich kann es gemäß einem weiteren, ggf. unabhängig zu beanspruchenden Aspekt der Erfindung grundsätzlich vorgesehen sein, dass ein von der Torsionswelle eingebrachtes Drehmoment über eine Anzahl von (ausschließlich) umfangsseitig an der Torsionswelle angeordneten Mitnahmeelementen auf die Mitnehmerwelle und von dieser unmittelbar auf ein darin aufgenommenes Werkzeug übertragen wird, wobei die Axialsicherung des Werkzeugs in der Mitnehmerwelle außerhalb dieses Drehmoment-Übertragungszugs durch axial wirkende Verriegelungselemente zwischen der Mitnehmerwelle und dem Werkzeug erfolgt.

Die Erfindung wird nachstehend anhand eines bevorzugten Ausführungsbeispiels sowie unterschiedlicher Varianten unter Bezugnahme auf die begleitenden Figuren näher erläutert.

**Figurenbeschreibung**

Fig. 1 zeigt den Längsschnitt eines chirurgischen Instruments dieser Gattung (einschließlich Handstück, Handstückschaft und Werkzeug) wie es auch aus dem Stand der Technik bekannt ist und welches als Referenz zur besseren Darstellung der Erfindung dienen soll,

Fig. 2 zeigt in Vergrößerung den proximalen Schaft - Endabschnitt eines Werkzeugs für das chirurgische Instrument nach Fig. 1,

Fig. 3 zeigt den Längsschnitt eines chirurgischen Instruments/Systems einschließlich Handstück, Handstückschaft und Werkzeug gemäß einem bevorzugten Ausführungsbeispiel der vorliegenden Erfindung,

Fig. 4a, 4b zeigen in Vergrößerung den proximalen Schaft - Endabschnitt eines erfindungsgemäßen Werkzeugs für ein chirurgisches Instrument nach Fig. 3,

Fig. 5 zeigt eine vergrößerte Längsschnittdarstellung des erfindungsgemäßen Instruments im Bereich des Werkzeugschlosses (der Werkzeugaufnahme / Werkzeugschloss),

Fig. 6 zeigt eine vergrößerte Querschnittdarstellung der Werkzeugaufnahme entlang der Schnittlinie A-A gemäß der Fig. 5 mit bereits eingesetztem Werkzeug,

Fig. 7a und 7b zeigen jeweils eine Längsschnittdarstellung einer ersten und zweiten erfindungsgemäßen Variante der Lagerung des Werkzeugs bzw. der Werkzeugaufnahme in einer Gegenüberstellung,

Fig. 8 zeigt in Vergrößerung den proximalen Werkzeugschaft - Endabschnitt gemäß einer zu Fig. 4 alternativen Variante,

Fig. 9 zeigt ein erfindungsgemäßes Beispiel einer Kodierungsmöglichkeit von unterschiedlichen Werkzeugschäften zur verwechslungssicheren Verwendung in unterschiedlichen Werkzeugaufnahmen (unterschiedlichen erfindungsgemäßen Handstückschäften/Handstücken),

Fig. 10 stellt zwei Beispiele für eine korrekte und eine inkorrekte Auswahl eines Werkzeugs für jeweils ein erfindungsgemäßes Instrumentenhandstück/Handstückschaft (mit bestimmter Aufnahme) entsprechend der erfindungsgemäßen Kodierung nach Fig. 9 dar,

Fig. 11 zeigt den Längsschnitt des erfindungsgemäßen chirurgischen Instrumentenhandstücks (nach Fig. 1) im Bereich des Werkzeugschlosses (Werkzeugaufnahme/-kupplung) ohne Werkzeug,

Fig. 12 und 13 beschreiben (chronologisch) den Montagevorgang zur Bereitstellung des Werkzeugschlosses (Werkzeugaufnahme) gemäß der Fig. 12

Fig. 14 zeigt den Querschnitt einer Kleeblattkupplung gemäß der Erfindung zur (lösbaren) (Drehmoment - Übertragungs - )Verbindung zwischen dem Werkzeugschloss (Werkzeugaufnahme oder Torsionswelle) und einer Abtriebswelle innerhalb des chirurgischen Instrumentenhandstücks für das Wechseln des distalen Handstückschafts,

Fig. 15 zeigt ein männliches und weibliches Teil der Kleeblattkupplung gemäß der Fig. 14 und

Fig. 16 zeigt schrittweise den Einführvorgang eines erfindungsgemäßen Werkzeugs in eine erfindungsgemäße Werkzeugaufnahme.

Das erfindungsgemäße chirurgische Instrument bzw. Instrumentensystem bestehend aus einem austauschbaren (Dreh-) Werkzeug, einem (universellen) Instrumentenhandstück und einem ggf. austauschbaren Handstückschaft (einschließlich darin gelagerter Torsionswelle) beinhaltet im Wesentlichen vier erfindungsgemäße Teilaspekte, welche im Rahmen dieser Erfindung unabhängig oder in Kombination miteinander beanspruchbar sind und welche nachfolgend im Einzelnen beschrieben werden. Dazu zählen

- die Gestaltung des proximalen Schaft - Endabschnitts des erfindungsgemäßen Werkzeugs des vorliegenden Instrumentensystems,
- die Schaffung einer Einstecksicherung in Form einer Werkzeugkodierung zur Vermeidung eines Werkzeug - Auswahl-/ Anwendungsfehlers,
- die Konstruktion des erfindungsgemäßen Werkzeugschlosses (oder auch Werkzeugaufnahme) innerhalb des Handstückschafts des Instrumentenhandstücks als Teil des Drehmomentübertragungszugs auf das Werkzeug (zur Kopplung von Werkzeug mit Torsionswelle innerhalb des Handstückschafts) sowie die Werkzeugschlosskonstruktion in seinem Bedienabschnitt und
- die Ausbildung einer Kupplung/drehmomentfesten Verbindung zwischen Werkzeugschloss (Werkzeugaufnahme) bzw. Torsionswelle innerhalb des Handstückschafts und einer Abtriebswelle innerhalb des Handstücks zur Ermöglichung der Auswechslung des distalen Handstückschafts (einschließlich des darin gelagerten Werkzeugschlosses und der Torsionswelle).

**Erfindungsgemäßes Werkzeug mit axial getrennter Drehmomentübertragung, Werkzeugeindrehung/Ausrichtung und axialer Verriegelung**

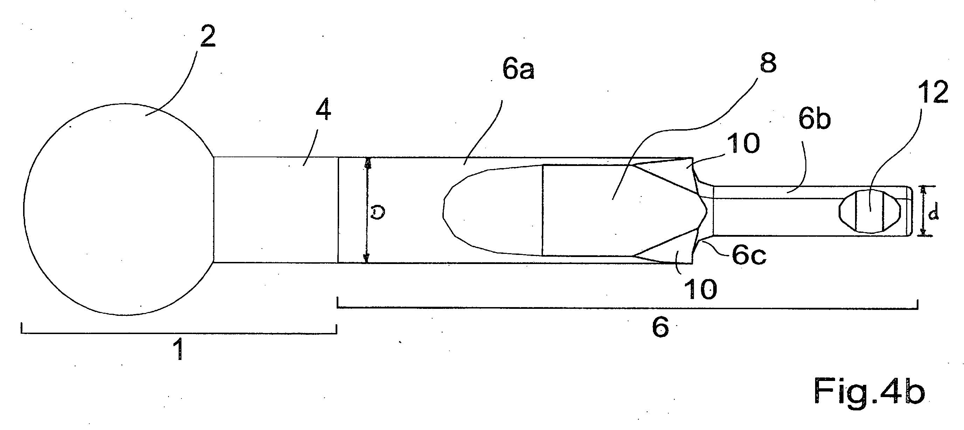

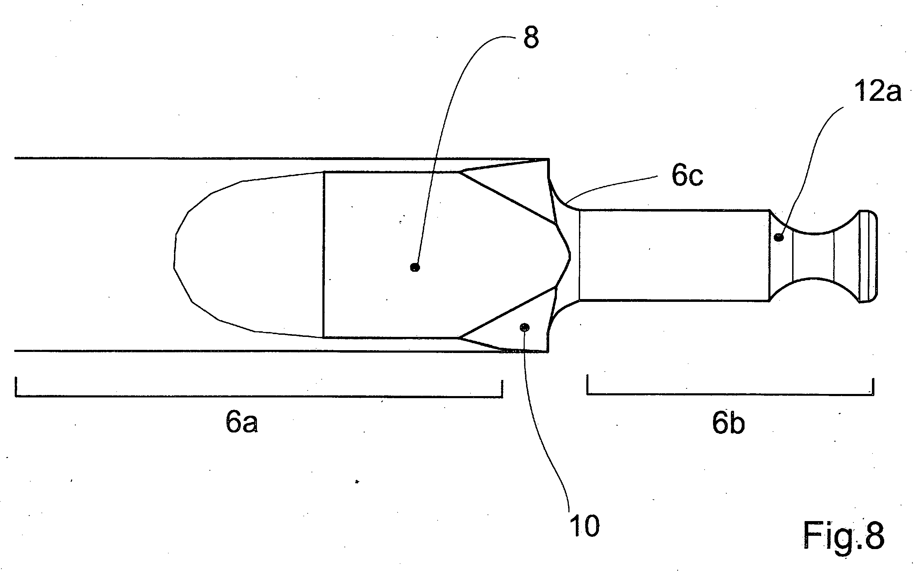

Gemäß der Fig. 4a, 4b und 8 besteht das erfindungsgemäße Werkzeug 1 grundsätzlich aus einem distalen (körperzugewandten) Eingriffssegment oder - abschnitt beispielsweise einem Bohr-, Fräs-, Schleif- oder Polierkopf 2, an den ein Werkzeugschaft 4 vorzugsweise stoffeinstückig (oder gelötet, geschweißt, gepresst, etc.) angesetzt ist, der sich in Richtung proximal (körperabgewandt) erstreckt. Dieser Werkzeugschaft 4 hat einen proximalen Endabschnitt 6 für das drehfeste Einsetzen des Werkzeugs 1 in eine Werkzeugaufnahme (Werkzeugschloss) eines chirurgischen Instrumentenhandstücks bzw. eines daran angeschlossenen Handstückschafts sowie für eine axiale Sicherung in der Werkzeugaufnahme.

Der Werkzeugschaft 1 ist hierfür in seinem proximalen Endabschnitt 6 in drei Funktionsbereiche unterteilt, welche axial voneinander (seriell) beabstandet sind und nachfolgend in chronologischer Reihenfolge ausgehend vom distalen Ende des Werkzeugschaft - Endabschnitts 6 (gemäß der Fig. 4a, 4b und 8 das linke Ende des proximalen Endabschnitts 6) beschrieben werden.

Wie aus den Fig. 4a, 4b und 8 zu entnehmen ist, besteht der gesamte Werkzeugschaft 4 gemäß der vorliegenden Erfindung zunächst aus einem distalen, im Wesentlichen unprofilierten Schaftabschnitt (schließt sich unmittelbar an das Eingriffssegment 2 an), sowie dem daran sich anschließenden proximalen Schaft-Endabschnitt 6, der wiederum in einen distalen Abschnitt 6a mit großem Schaftdurchmesser sowie einer Außenprofilierung und einem proximalen Abschnitt 6b mit kleinem Schaftdurchmesser seriell unterteilt ist. Das Durchmesserverhältnis zwischen großem und kleinem Schaftdurchmesser D:d innerhalb des proximalen Schaft-Endabschnitts 6 beträgt in etwa 2:1. D.h., der kleine Schaftdurchmesser d ist im Wesentlichen kleiner/gleich der Hälfte des großen Schaftdurchmessers D. Konkreter ausgedrückt soll gelten: d <= 0,6 D. Dabei verjüngt sich der große Schaftdurchmesser D nicht kontinuierlich zum kleinen Schaftdurchmesser d sondern es bildet sich ein Radialabsatz 6c zwischen beiden Schaftabschnitten 6a, 6b unterschiedlichen Durchmessers ggf. mit einem geringen Innenradius zur Verminderung einer Kerbwirkung.

Im Bereich des Radialabsatzes 6c ist der großdurchmessrige Schaftabschnitt 6a gemäß der Fig. 4a mit zwei diametral sich gegenüberliegenden Angriffsflächen 8 oder -ebenen ausgebildet (sogenannter Zweiflach), die sich keilförmig in Richtung hin zum Radialabsatz 6c annähern und zur Einleitung eines Drehmoments in den Werkzeugschäft 4 dienen. Diese Angriffsebenen 8 können insbesondere durch Anschleifen/-fräsen oder durch Pressen/Schmieden des zunächst unprofilierten, runden Werkzeugschafts 4 gebildet sein. An den axialen Seitenkanten jeder Angriffsfläche 8 sind (im Bereich des Radialabsatzes 6c) zusätzliche Abgleitflächen oder - ebenen 10 ausgebildet (vorzugsweise hergestellt wie die Angriffsflächen 8), die jeweils in einem Winkel zur zugehörigen Angriffsfläche 8 ausgerichtet sind und ausgehend von einem axialen Mittenbereich jeder Angriffsebene-Seitenkante.in Richtung hin zum Radialabsatz 6c keilförmig zulaufen. Dadurch entsteht im Bereich des Radialabsatzes 6c ein Schaftprofil mit sechs Flächen bestehend aus den zwei diametral gegenüberliegenden Angriffsebenen 8 (Zweiflach) sowie in Umfangsrichtung zu beiden Seiten jeder Angriffsebene 8 jeweils einer Abgleit- oder Eindrehebene 10, welche die entsprechende Seitenkante der jeweiligen Angriffsfläche 8 im Bereich des Radialabsatzes 6c bricht und damit die Breite der jeweiligen Angriffsebene 8 in Richtung hin zum Radialabsatz 6c zunehmend verringert.

Am proximalen Ende des kleindurchmessrigen Schaft-Endabschnitts 6b sind gemäß der Fig. 4a und 4b zwei diametral am Schaftumfang angeordnete Kerben oder Taschen 12 ausgeformt (vorzugsweise eingefräst), wodurch sich axial wirkende Hinterschneidungen an der Schaftoberfläche bilden. Alternativ zu diesen Kerben 12 ist es aber gemäß der Fig. 8 auch möglich, am proximalen Ende des kleindurchmessrigen Schaft-Endabschnitts 6c eine umlaufende Nut 12a auszudrehen, deren Nutentiefe im Wesentlichen der Kerbentiefe gemäß der Fig. 4a, 4b entspricht. Diese Kerben 12 oder Umfangsnut 12a dienen zur axialen Verriegelung des Werkzeugschafts 4 innerhalb einer Werkzeugaufnahme, wie sie nachfolgend noch beschrieben wird.

Durch die vorstehend beschriebene Schaftkonstruktion insbesondere im profilierten Werkzeugschaft-Endabschnitt 6 lassen sich gegenüber dem Stand der Technik gemäß der Fig. 1 und 2 einige Vorteile erzielen, welche zur Steigerung des maximal übertragbaren Drehmoments von der Torsionswelle innerhalb des Handstückschafts auf das Werkzeug 1 beitragen:

- Durch die prinzipielle Trennung von axialer Sicherung/Verriegelung und Drehmomentmitnahme (mit dazwischen angeordneter Eindrehhilfe) in zwei (ggf. drei) axial beabstandete Schaftabschnitte können diese Funktionsabschnitte unabhängig voneinander optimiert werden.

- Entscheidend dabei ist, dass der Funktionsabschnitt "Verriegelung" 6b bezüglich des Funktionsabschnitts "Drehmomentmitnahme" 6a proximal angeordnet ist. Dies ermöglicht es, den Verriegelungsabschnitt 6b, der nicht auf Torsion/Drehmoment belastet wird, gegenüber dem Drehmomentmitnahmeabschnitt 6a kleindurchmessrig auszugestalten und dadurch den Radialabsatz 6c zu bilden.

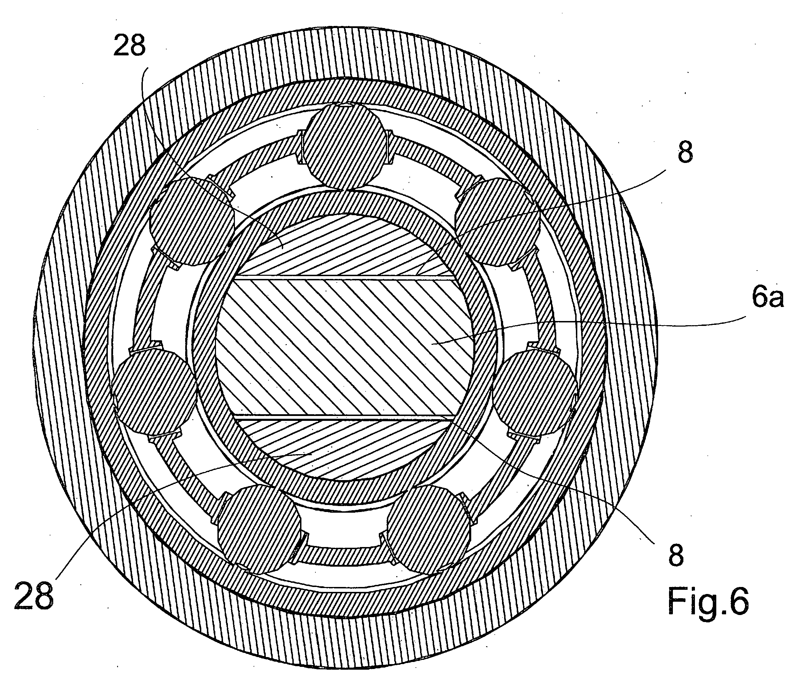

- Der Radialabsatz 6c wiederum erlaubt es, distal zum kleindurchmessrigen Verriegelungsabschnitt 6b zwei Angriffs- oder Drehmomentübertragungsebenen 8 mit größerer axialer Länge auszuformen (abzufräsen), um dadurch deren jeweilige Fläche gegenüber dem Stand der Technik zu vergrößern. Durch den Radialabsatz 6c kann zudem (wie insbesondere in der Fig. 6 dargestellt ist) in dessen Bereich so viel Schaftmaterial zur Ausbildung der Angriffsebenen 8 abgetragen werden, dass sich der verbleibende Schaftdurchmesser (im Radialabsatzbereich) zwischen den beiden (keilförmigen) Ebenen 8 nahezu halbiert. Damit nähert sich gemäß der Fig. 6 der zur Drehmomentübertragung nutzbare Durchmesser Dm dem großen Schaftdurchmesser D an. Wird somit der so ausgebildete Zweiflach in einen Axialspalt einer Mitnahmewelle geschoben (wird nachfolgend noch beschrieben), ergibt sich gemäß der Fig. 6 wieder ein Vollkreis mit optimalem Hebelverhältnis zur Drehmomentübertragung.

- Bisher wurde die axiale Verriegelung gemäß der Fig. 1 und 2 zwischen dem Funktionsabschnitt "Drehmomentmitnahme" und dem WerkzeugEingriffssegment angeordnet, wodurch sich die maximal mögliche axiale Ausdehnung des Funktionsabschnitts "Drehmomentmitnahme" beschränkt. Durch die dadurch sich ergebende steilere Keilform der beiden Angriffsebenen wirken große axiale Kräfte auf die axiale Verriegelung. Zudem wird der im Drehmomentfluss angeordnete Verriegelungsabschnitt materiell geschwächt. Nunmehr ist es vorgesehen, die axiale Verriegelung 6b proximal zur Drehmomentmitnahme 6a (also nicht zwischen Drehmomentmitnahme und Eingriffssegment) außerhalb des Drehmomentflusses zu platzieren. Damit kann die Keilform der beiden Angriffsebenen 8 insgesamt abgeflacht werden (unter größerer axialer Ausdehnung), wodurch sich axiale Kräfte bei der Drehmomentübertragung verringern. Somit kann der benötige (radiale)-Bauraum für die axiale Verriegelung 6b verkleinert werden (kleiner Werkzeugschaftdurchmesser d möglich).

- Schließlich wird durch die Ausbildung des Radialabsatzes 6c zwischen den Funktionsabschnitten "Drehmomentübertragung" 6a und "axiale Verriegelung" 6b die Möglichkeit der Anordnung zusätzlicher Abgleitebenen 10 als Eindrehhilfe im Funktionsabschnitt "Drehmomentübertragung" 6ä gegeben. Diese Abgleitebenen 10 sind jeweils zu beiden axialen Seiten der beiden Angriffsebenen 8 ebenfalls keilförmig ausgebildet und brechen dabei die Seitenkanten der Angriffsebenen 8 im Bereich des Radialabsatzes 6c - d.h. sie sind in einem Winkel zur jeweiligen Angriffsebene 8 ausgerichtet. Diese Abgleitebenen 10 dienen dazu, den Werkzeugschaft 4 bei Einführen in eine Werkzeugaufnahme des Handstücks in Umfangsrichtung auszurichten, derart, dass die beiden Angriffsebenen 8 korrekt in die Werkzeugaufnahme geleitet werden.

## Erfindungsgemäßes Werkzeug mit Werkzeugkodierung

Wie vorstehend bereits ausgeführt wurde, besteht ein wesentliches Merkmal der vorliegenden Erfindung darin, den Funktionsabschnitt "axiale Verriegelung" 6b proximal zum Funktionsabschnitt "Drehmomentübertragung" 6a anzuordnen. Darüber hinaus kann der erfindungsgemäße Werkzeugschaft 4 auch noch alle weiteren Merkmale gemäß der vorstehenden Beschreibung aufweisen, wobei diese jedoch für den nachfolgenden Erfindungsaspekt "Werkzeugkodierung" nur optional sind.

Grundsätzlich existiert ein Anwenderwunsch, Behandlungsfehler insbesondere infolge falscher chirurgischer Werkzeuge zu minimieren oder auszuschließen. Dies lässt sich beispielsweise durch optische Kennungen an den einzelnen Werkzeugen bewerkstelligen, wobei jedoch in diesem Fall der Faktor "Mensch" als Fehlerquelle nicht ausgeschlossen werden kann. D.h., in der Praxis können optische Kennungen übersehen oder missverstanden/verwechselt werden, sodass es bei der Auswahl eines bestimmten Werkzeugs zu Fehlern kommen kann, die erst bei deren Einsatz ggf. zu spät erkannt werden. Diese Fehlerquelle ist umso bedeutender, je mehr verschiedene Werkzeuge im Rahmen eines Instruments/Instrumentensystems einem universellen Handstück zugeordnet werden können. In diesem Fall ist es daher vorteilhaft und wünschenswert, wenn für bestimmte chirurgische Einsatzzwecke in Abhängigkeit eines bestimmten, an das Universalhandstück angeschlossenen Handstückschafts (mit innen gelagerter Torsionswelle) nur eine begrenzte Zahl an Werkzeugen eingesetzt werden kann.

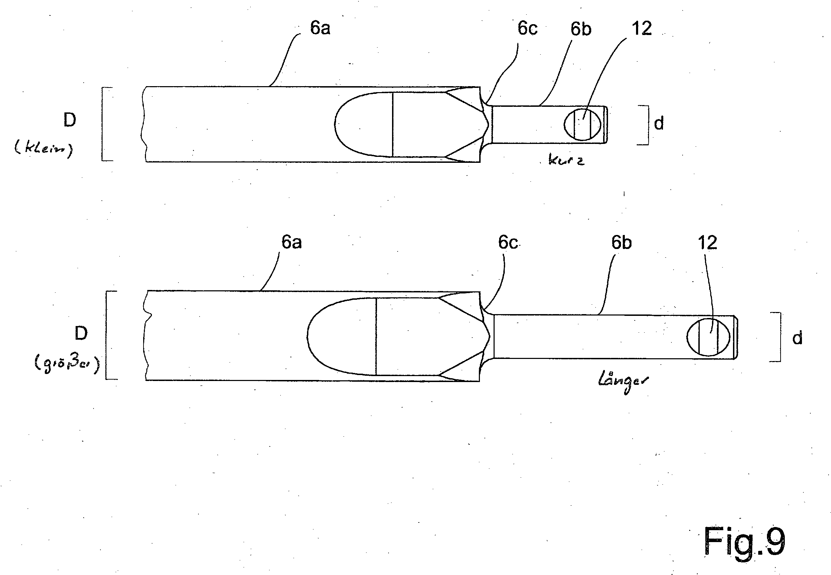

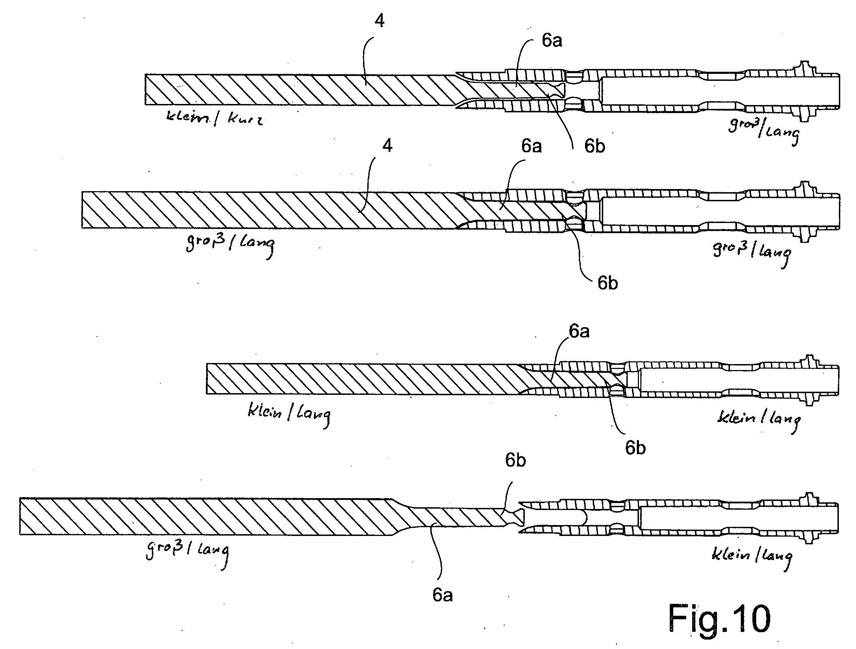

In den Fig. 9 und 10 ist eine vorteilhafte Variante einer erfindungsgemäßen Werkzeugkodierung dargestellt, durch die eine Falschauswahl eines Werkzeugs vermeidbar ist.

Die drehmomentfreie Anordnung des Funktionsabschnitts "axiale Verriegelung" 6b proximal zum Funktionsabschnitt "Drehmomentübertragung" 6a bietet die grundsätzlichen (optionalen) Möglichkeiten, die axiale Länge und/oder den (kleindurchmessrigen) Schaftdurchmesser d dieses Funktionsabschnitts 6b zu verändern, ohne dass hierdurch der Funktionsabschnitt "Drehmomentübertragung" 6a (nachteilig) beeinflusst wird. D.h. es wird hierdurch möglich, wenigstens zwei (oder mehrere) unterschiedliche axiale Abschnittslängen (d.h. axialer Abstand zwischen Radialabsatz 6c und radiale Tasche/Umfarigsnut 12/12a bzw. axial wirkende Hinterschneidung) und/oder wenigstens zwei (oder mehrere) unterschiedliche (kleindurchmessrige) Schaftdurchmesser d vorzusehen (bzw. zu kombinieren), die nur mit entsprechend dimensionierten Werkzeugaufnahmen funktionell zusammenwirken können.

Beispielsweise sind in der Fig. 9 die beiden Kombinationen "kurzer Verriegelungsabschnitt" mit "kleinerem Schaftdurchmesser" und "langer Verriegelungsabschnitt" mit "größerem Schaftdurchmesser" bezüglich des Funktionsabschnitts "Verriegelung" 6b gezeigt. Demzufolge wird die Werkzeugaufnahme (welche nachfolgend noch detailliert beschrieben wird) gemäß der Fig. 10 prinzipiell so ausgebildet, dass zwar der kleinere Schaftdurchmesser auch in die Aufnahme für den größeren Schaftdurchmesser für eine Drehmomentübertragung einführbar ist, jedoch keine axiale Verriegelung stattfindet und daher das Werkzeug 1 bei Überprüfung des korrekten Werkzeugsitzes wieder herausgezogen werden kann (obere Darstellung). Wird indessen in diese Werkzeugaufnahme der größere Schaftdurchmesser eingeführt, findet eine axiale Verriegelung statt (zweite Darstellung von oben). Im Gegenzug erlaubt eine Aufnahme für den kleineren Schaftdurchmesser erst gar kein Einführen des größeren Schaftdurchmessers (untere Darstellung), wohingegen der kleinere Schaftdurchmesser eingeführt und axial verriegelt werden kann (zweite Darstellung von unten).

An dieser Stelle sei darauf hingewiesen, dass die Länge und der Schaftdurchmesser des Funktionsabschnitts "axiale Verriegelung" 6b nur zwei Kodierparameter darstellen, die besonders einfach erkennbar sind, die jedoch auch durch andere Parameter ersetzt oder ergänzt werden können. Beispielsweise kann die Umfangsposition der Taschen 12 bezüglich der beiden Angriffsebenen 8 dazu dienen, ein Verriegeln nur bei der richtigen vorbestimmten Relativposition (bei entsprechend korrekter Ausrichtung der Angriffsebenen 8 zur Werkzeugaufnahme) zu ermöglichen. Auch die Form der Taschen 12 ist veränderbar, derart, dass nur kompatible Formen auf Seiten der Aufnahme eine sichere axiale Verriegelung ergeben. Schließlich kann der Abschnitt "axiale Verriegelung" 6b mit einer zusätzlichen Form ausgestaltet sein (nicht dargestellt), die nach dem "Schlüssel-Schlüsselloch-Prinzip" mit einer entsprechenden Form in der Werkzeugaufnahme zusammenwirkt, um ein Einführen der Werkzeugschafts 4 zu erlauben (z.B. Nut-Feder-Anordnung).

**Handstückschaft mit erfindungsgemäßer Werkzeugaufnahme (oder auch Werzeugschloss)**

Eine in einem Handstückschaft unterzubringende Werkzeugaufnahme insbesondere für ein (Einheits-) Werkzeug gemäß dem vorstehend beschriebenen ersten und/oder zweiten Aspekt der Erfindung hat mehrere Anforderungen zu erfüllen, die im Wesentlichen die Folgenden umfassen:

- Geringe radiale Abmessungen zur Ermöglichung von deren Unterbringung in einem bekanntlich engen Handstückschaft.
- Übertragung eines ausreichenden Arbeitsdrehmoments auf das Werkzeug.
- Ergonomisch günstige und einfache manuelle Betätigung zumindest zur Freigabe des darin eingesetzten Werkzeugs und vorzugsweise automatisches Verriegeln des Werkzeugs (halbautomatische Werkzeugaufnahme).
- Sicherung von Werkzeugaufnahme und Werkzeug im Betrieb gegen selbsttätige Demontage (etwa bei Vibrationen, Stößen und/oder Schlägen) zur Erhöhung der Instrumentenzuverlässigkeit.
- Einfaches und zerstörungsfreies Montieren und Demontieren der Aufnahme beispielsweise zu Reinigungs- oder Wartungszwecken.

Der Zweck einer solchen Aufnahme innerhalb des Handstückschafts besteht dabei grundsätzlich darin, die Werkzeugaufnahme beliebig (möglichst) weit nach distal zu verlagern und damit den Werkzeugschaft auf eine bezüglich der zu erwartenden Biegekräfte während des Werkzeugeinsatzes optimale (Einheits-) Länge zu beschränken. Damit kann ein solches (Einheits-) Werkzeug für unterschiedliche Schaftlängen und Schaftformen vorgesehen sein, wobei die Schaftstrecke zwischen Handstück und Werkzeugaufnahme durch eine im Handstückschaft gelagerte ggf. biegeflexible oder starre Torsionswelle überbrückt wird.

Die in der Fig. 1 schematisch dargestellte, bekannte Werkzeugaufnahme hat zwar hinsichtlich ihrer räumlichen (insbesondere radialen) Abmessungen das Potential, innerhalb eines per se bekannten Handstückschafts bekannter Bauform verbaut zu werden. Indessen stellen insbesondere die Querstifte zur Verbindung des Aufnahmerohrs mit der Torsionswelle sowie zur drehfesten Kopplung des Aufnahmerohrs mit dem darin gelagerten, auf das Werkzeug einwirkenden Drehmoment-Übertragungsbolzen jeweils eine Schwachstelle im Drehmoment-Übertragungszug dar, wie dies eingangs bereits beschrieben wurde.

Wie die Fig. 1 im Detail nämlich zeigt, ist der innen liegende Drehmoment-Übertragungsbolzen mittels des einen (dünnen) Querstifts, der in einer darin ausgebildeten Längs-Querbohrung lagert, zumindest mit dem äußeren Aufnahmerohr gekoppelt. Ein für alle Anwendungszwecke geeignetes maximales Drehmoment ist mit einem derartigen (dünnen) Querstift nicht sicher übertragbar. Die Bohrungen für den Querstift schwächen zusätzlich die ohnehin sehr kleinen zu verbindenden Bauteile, nämlich Aufnahmerohr und Bolzen. Außerdem ist, wie dies vorstehend bereits ebenfalls geschildert wurde, ein weiterer Querstift zur Kopplung des Aufnahmerohrs mit der Eingangs- bzw. Torsionswelle vorgesehen, der die gleichen Probleme verursacht. Unabhängig davon ist das Verbinden von demnach drei Bauteilen mittels der genannten Querstifte fertigungs- und montagetechnisch besonders bei kleinen Dimensionen wie bei den gattungsgemäßen Handstückschäften einschlägig bekannter Bauart sehr schwierig und zeitraubend. Daher ist es wünschenswert, eine Werkzeugaufnahme insbesondere für ein Werkzeug mit einem vorstehend beschriebenen Aufbau bereit zu stellen, welche diese Probleme löst.

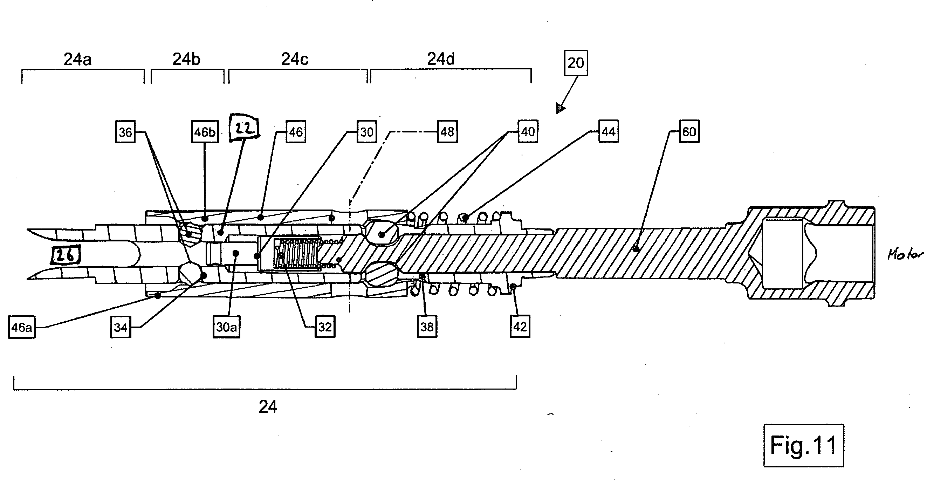

Die Fig. 11 zeigt ein bevorzugtes Ausführungsbeispiel einer solchen erfindungsgemäßen Werkzeugaufnahme 20, deren Bauteile nachfolgend im Einzelnen sowie in deren Zusammenwirken mit dem eingangs beschriebenen Werkzeug 1 erläutert werden.

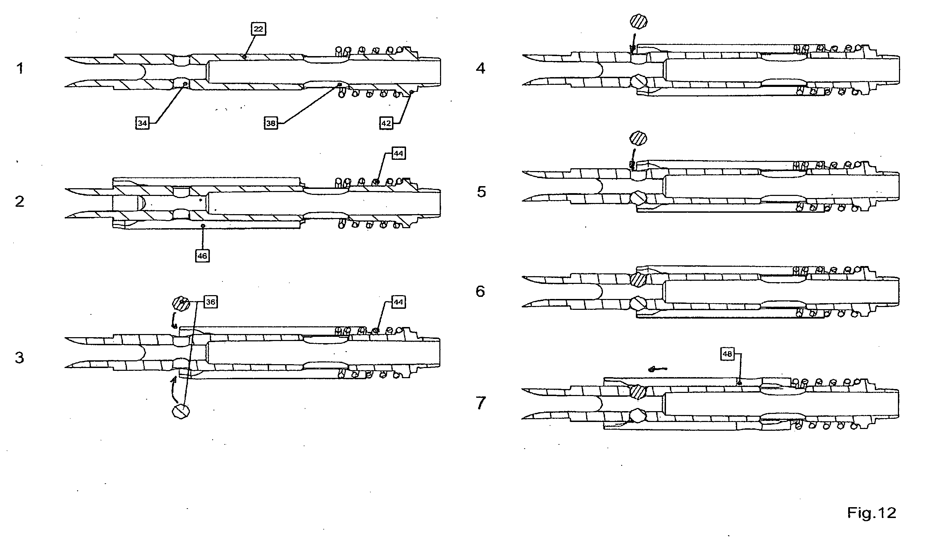

Zunächst hat die erfindungsgemäße Werkzeugaufnahme oder Werkzeugschloss 20 gemäß dem bevorzugten Ausführungsbeispiel der vorliegenden Erfindung ein radial inneres Werkzeug-Aufnahmerohr (nachfolgend als Mitnehmerwelle bezeichnet) 22 (siehe auch Fig. 12) mit einem an ihrem distalen Ende längsgeschlitzten (schnabelförmigen) distalen Drehmoment-Übertragungs- und Verriegelungsabschnitt 24, der in seinem geschlitzten Drehmoment-Übertragungsbereich 24a (siehe auch die Fig. 6) einen an den großdurchmessrigen Werkzeugschaftabschnitt 6a angepassten Außendurchmesser und in seinem daran sich anschließenden Verriegelungsbereich 24b einen an den kleindurchmessrigen Werkzeugschaftabschnitt 6b angepassten Innendurchmesser hat. Der Längsschlitz 26 bildet dabei ein Schlitzweite, in die der Werkzeugschaft 1 im Bereich der beiden keilförmigen Angriffsebenen 8 eingeschoben werden kann (siehe Fig. 6), sodass die werkzeugseitigen Angriffsebenen 8 an den dadurch entstandenen schnabelförmigen Axialvorsprüngen 28 des Drehmoment-Übertragungsbereichs 24a flächig anliegen und dabei gemeinsam ein geschlossenes Voll-Rundprofil bilden (siehe Fig. 6).

An den Verriegelungsbereich 24b schließt sich proximal ein zylindrischer Bolzenaufnahmeabschnitt 24c mit gegenüber dem Verriegelungsbereich 24b größerem Innendurchmesser (der darin gelagerte Bolzen 30 wird nachfolgend als Nachfahrelement bezeichnet) unter Ausbildung eines inneren Radialabsatzes an, der als Axialanschlag für das Nachfahrelement 30 in Richtung distal dient. Das Nachfahrelement 30 hat hierfür einen distalen Abschnitt 30a mit einem Außendurchmesser entsprechend dem kleindurchmessrigen Werkzeugschaftabschnitt "Verriegelung" 6b, der somit in den Verriegelungsbereich 24b der Mitnehmerwelle 22 eingefahren werden kann sowie einen proximalen Abschnitt mit größerem Außendurchmesser 30b, an dem das Nachfahrelement 30 gleitend in der Mitnehmerwelle 22 geführt ist. Zwischen den beiden Abschnitten 30a, 30b des Nachfahrelements 30 bildet sich ebenfalls ein äußerer Ringabsatz aus, der mit dem inneren Ringabsatz der Mitnehmerwelle 22 in Richtung distal zusammenwirkt.

Schließlich ist in dem Aufnahmeabschnitt 24c für das Nachfahrelement 30 eine Nachfahrfeder 32 angeordnet, welche das Nachfahrelement 30 in Richtung distal vorspannt und so gegen den inneren Ringabsatz in der Mitnehmerwelle 22 drückt. In dieser Position ist der kleiner durchmessrige distale Abschnitt 30a des Nachfahrelements 30 vollständig in den Verriegelungsbereich 24b der Mitnehmerwelle 22 eingefahren.

An dieser Stelle sei erwähnt, dass die Mitnehmerwelle 22 in deren Verriegelungsbereich 24b eine Anzahl (mindestens eine) von gleichmäßig umfangsbeabstandeten radialen Durchgangsbohrungen 34 hat, die zur Aufnahme von Verriegelungskugeln 36 für das eingesetzte Werkzeug 1 dienen, wie dies nachfolgend noch beschrieben wird.

In proximaler Verlängerung des Aufnahmeabschnitts 24c für das Nachfahrelement 30 bildet die Mitnehmerwelle 22 einen Kopplungs-/Einsteckabschnitt 24d für eine Antriebs-/Torsionswelle 60, welche in einem in der Fig. 11 nicht dargestellten Handstückschaft (siehe beispielsweise Fig. 3) eines (universellen) Handstücks drehbar gelagert ist.

In diesem Einsteckabschnitt 24d hat die Mitnehmerwelle 22 ebenfalls eine Anzahl (wenigstens eine) gleichmäßig umfangsbeabstandeter sowie auf einer Kreisebene liegender Radialöffnungen oder Durchbrüche 38 von in etwa ovalem Querschnitt, der sich jeweils in Axialrichtung der Mitnehmerwelle 22 ausdehnt. Diese Radialöffnungen 38 dienen zur Aufnahme von vorzugsweise ovalen Wälzkörpern 40 (nachfolgend als Mitnahmeelemente bezeichnet), über welche die Mitnehmerwelle 22 dreh- und axialfest mit der eingesteckten Torsionswelle 60 gekoppelt ist, was nachfolgend noch näher beschrieben wird. Dabei sei noch erwähnt, dass anstelle ovaler (zylindrischer) Wälzkörper mit abgerundeten Stirnseiten auch Kugeln verwendet werden können.

Am proximalen Ende des Einsteckabschnitts 24d hat die Mitnehmerwelle 22 ferner einen umlaufenden Radialvorsprung 42, der als Federsitz einer äußeren Schließfeder 44 dient.

Um die Mitnehmerwelle 22 herum ist eine Schließhülse 46 dreh- und axial verschiebbar gelagert. Diese hat einen distalen Kugel-Freigabebereich 46a mit großem Innenradius und einen daran proximal sich anschließenden Kugel-Sperrbereich 46b mit kleinem Innenradius, der auch gleitend an der Außenseite der Mitnehmerwelle 22 geführt ist.

In einem proximalen Endabschnitt der Schließhülse 46 sind eine Anzahl (vorzugsweise zwei) von radialen Durchgangsöffnungen 48 mit längsovalem (oder runden) Querschnitt ausgeformt, die zur Befüllung der in der Mitnehmerwelle 22 vorgesehenen Durchbrüche 38 mit den ovalen/tonnenförmigen, abgerundeten Wälzkörpern 40 dienen. Jede dieser ovalen (größere Länge als Breite) Durchgangsöffnungen 48 der Schließhülse 46 ist am Innenumfang der Schließhülse 46 zu einer Aufnahmetasche verlängert, sodass die Schließhülse 46 die bereits eingelegten Wälzkörper/Mitnahmeelemente 40 axial überfahren und gegen ein Herausfallen verriegeln kann. Gleichzeitig sind die Aufnahmetaschen so geformt, dass die Schließhülse 46 bezüglich der Mitnehmerwelle 22 um einen bestimmten Winkel gedreht werden kann, sodass auch bei einem Zurückfahren der Schließhülse 46 in eine axiale Freigabeposition die Wälzkörper 40 und auch die Verriegelungskugeln gemäß nachfolgender Beschreibung nicht mehr herausfallen können.

Schließlich ist axial zwischen der Schließhülse 46 und dem äußeren Radialvorsprung 42 der Mitnehmerwelle 22 die Schließfeder 44 angeordnet, welche die Schließhülse 46 in Richtung distal in eine axiale Verriegelungsposition drückt.

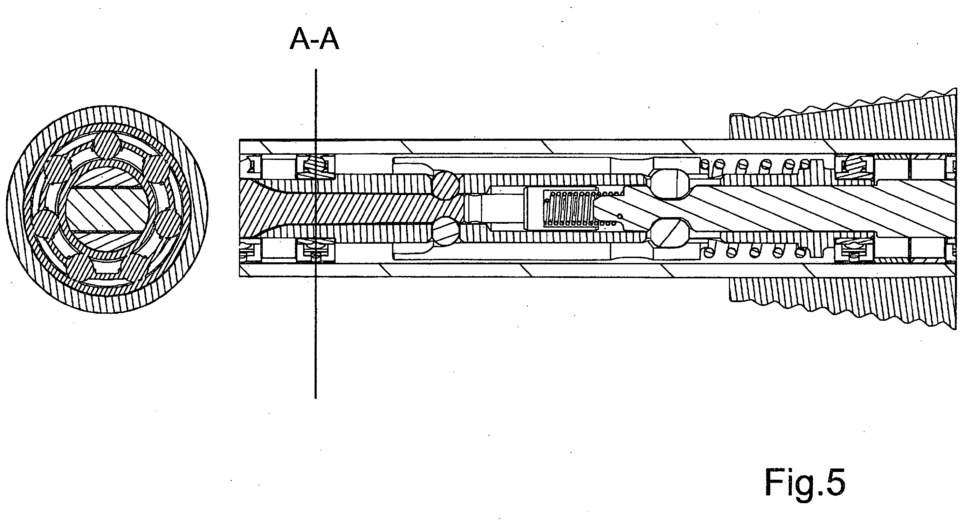

Die Montage und Funktionsweise der erfindungsgemäßen Werkzeugaufnahme 20 wird nachfolgend anhand der Fig. 11 bis 13 in Verbindung mit der Fig. 5 näher erläutert.

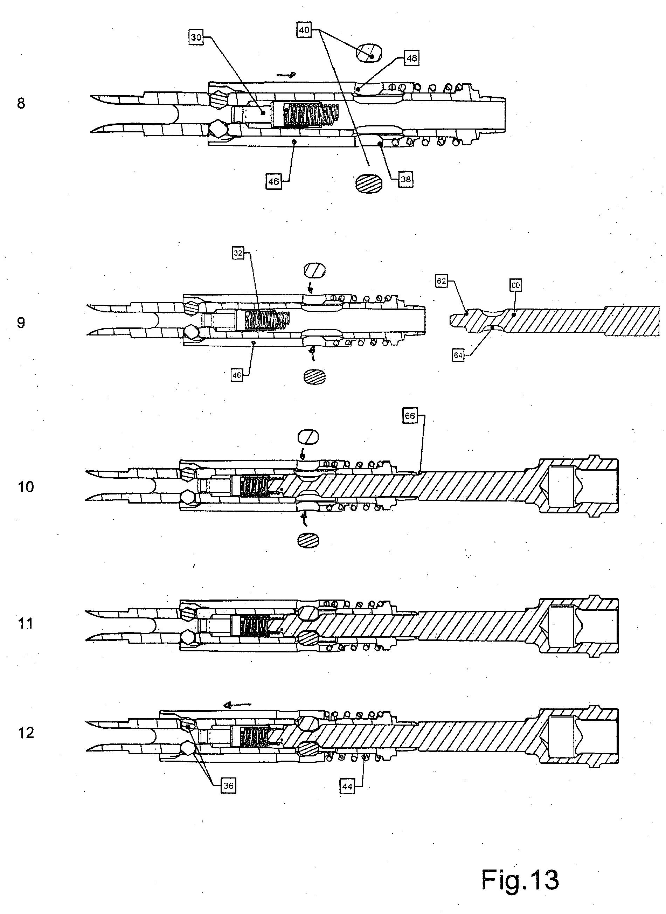

Gemäß der Fig. 12 und 13 wird für eine Montage der Werkzeugaufnahme 20 an einer Torsionswelle 60 zunächst die äußere Schließfeder 44 über die Mitnehmerwelle 22 gestreift und anschließend die Schließhülse 46 auf die Mitnehmerwelle 22 aus der Richtung distal aufgesteckt, sodass die äußere Schließfeder 44 zwischen der Schließhülse 46 und dem äußeren Radialvorsprung 42 an der Mitnehmerwelle 22 zu liegen kommt (siehe Abbildungen 1 und 2 der Fig. 12).

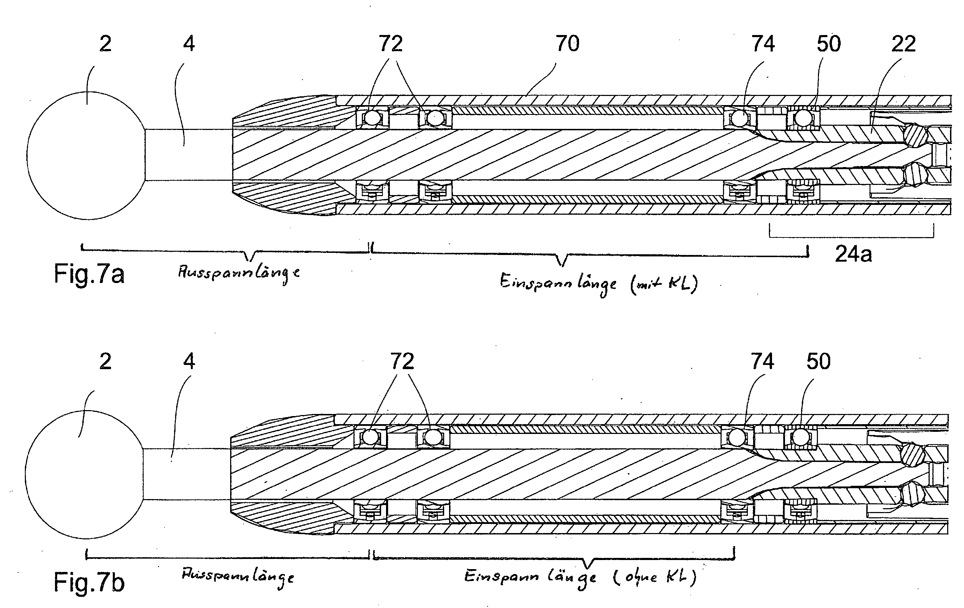

Anschließend wird die Schließhülse 46 gegen die äußere Schließfeder 44 in ihre axiale Befüll- bzw. Freigabeposition gedrückt, wodurch die Durchgangsbohrungen 34 im Verriegelungsbereich 24b der Mitnehmerwelle 22 freigelegt werden. Jetzt können in diese Durchgangsbohrungen 34 die Verriegelungskugeln 36 über eine Montagenut an der Innenseite der Schließhülse 46 eingesetzt werden, die radial nach Innen vorragen (siehe Abbildungen 3 bis 6 der Fig. 12). Abschließend kann die Schließhülse 46 freigegeben werden, wodurch diese durch die äußere Schließfeder 44 axial in Kugelsperrposition verschoben wird, in der die Schließhülse 46 die Verriegelungskugeln 36 überfährt und somit gegen ein radiales Herausfallen sichert. Die Kugeln 36 dienen dabei gleichzeitig als Axialanschlag für die Schließhülse 46, die hierfür an ihrem distalen Innenumfang einen kleinen Innen-Radialabsatz hat, der in Sperr-Verriegelungsposition der Schließhülse 46 axial an den Verriegelungskugeln 36 anliegt (siehe Abbildung 7 der Fig. 12). Damit ist die Vormontage der erfindungsgemäßen Werkzeugaufnahme 20 abgeschlossen.

In der Fig. 13 ist nunmehr die Montage der Werkzeugaufnahme 20 an einer Torsionswelle 60 dargestellt.

Zunächst wird in die Mitnehmerwelle 22 aus der Richtung proximal das Nachfahrelement 30 und daraufhin die innere Nachfahrfeder 32 eingesetzt, wobei der distale Abschnitt 30a des Nachfahrelements 30 an den Verriegelungskugeln 36 axial anliegt. Anschließend wird die Torsionswelle 60 in die Mitnehmerwelle 22 aus der Richtung proximal eingesteckt. Die Torsionswelle 60 bildet an ihrem distalen Ende einen radialen Absatz 62 als Federsitz für die bereits eingelegte innere Nachfahrfeder 32. Des Weiteren hat die Torsionswelle 60 an ihrem distalen Endabschnitt eine Anzahl von gleichmäßig umfangsbeabstandeten Außentaschen 64 zur Aufnahme der Mitnahmeelemente (ovale Wälzkörper) 40. Schließlich bildet die Torsionswelle 60 umfangsseitig optional einen Wellenabsatz 66 als Axialanschlag für die Mitnehmerwelle 22.

Sobald die Mitnehmerwelle 22 am optionalen Axialanschlag 66 der Torsionswelle 60 anliegt, überlappen sich die radialen Außentaschen 64 der Torsionswelle 60 genau mit den proximalen Durchbrüchen 38 der Mitnehmerwelle 22 sowie den Befüllöffnungen 48 der in die axiale Befüll-/Freigabeposition geschobenen Schließhülse 46 (siehe die Abbildungen 8 bis 10 der Fig. 13). Jetzt können die ovalen Mitnahmeelemente 40 über die Befüllöffnungen 48 der Schließhülse 46 in die Durchbrüche 38 der Mitnehmerwelle 22 sowie die Außentaschen 64 der Torsionswelle 60 eingesetzt werden (siehe Abbildung 11 der Fig. 13). Abschließend wird die Schließhülse 46 freigegeben, die selbsttätig durch die Schließfeder 44 in die Verriegelungsposition axial in Richtung distal verschoben wird, in welcher die Verriegelungskugeln 36 wie auch die Mitnahmeelemente 40 von der Schließhülse 46 überfahren und somit gegen ein radiales Herausfallen gesichert werden. Abschließend wird die Schließhülse 46 um einen bestimmten Winkel bezüglich der Mitnehmerwelle 22 gedreht. Dadurch wird verhindert, dass auch bei einem erneuten Zurückziehen der Schließhülse 46 in die Kugel-Freigabeposition während eines normalen Betriebs die Kugeln 36 und vorzugsweise auch die Mitnahmeelemente 40 über die Befüllöffnungen 48 der Schließhülse 46 unbeabsichtigt herausfallen können. D.h. die Axialposition der Schließhülse 46 für ein Befüllen mit den Mitnahmeelementen 40 sowie für ein radiales Freigeben der Kugeln 36 beim Einstecken eines Werkzeugs 1 ist vorzugsweise gleich. Die Winkelposition der Schließhülse 46 bezüglich der Mitnehmerwelle 22 in der Befüllposition ist jedoch zur Winkelposition in der Freigabeposition unterschiedlich.

Damit ist der Montagevorgang der Werkzeugaufnahme 20 an der Torsionswelle 60 abgeschlossen.

Wie die vorstehende Beschreibung des Montagevorgangs verdeutlicht, sind radial äußere Mitnahmeelemente 40 vorzugsweise in Form von ovalen Wälzkörpern für eine Drehmomentübertragung von der Torsionswelle 60 auf die Mitnehmerwelle 22 vorgesehen. Diese Elemente besitzen folglich eine große wirksame Kraftangriffsfläche und können daher erhebliche Drehmomente übertragen, ohne abzuscheren. Gleichzeitig dienen die Mitnahmeelemente zur Axialsicherung der Werkzeugaufnahme auf der Torsionswelle. Durch die radial äußere Positionierung wird zudem ein maximaler Hebel zur Drehmomentübertragung erreicht.

Das Drehmoment wird erfindungsgemäß nicht wie im genannten Stand der Technik über das Nachfahrelement (Bolzen) 30 sondern unmittelbar über die Mitnehmerwelle 22 auf den Werkzeugschaft 4 übertragen. Dadurch reduzieren sich die in den Drehmoment-Übertragungszug eingebundenen Bauteile, wodurch die Montage insgesamt vereinfacht wird.

Die Funktionsweise der erfindungsgemäßen Werkzeugaufnahme 20 wird nachstehend anhand der Fig. 5, 7a, 7b und 16 näher erläutert.

Zunächst ist darauf hinzuweisen, dass die Werkzeugaufnahme 20 innerhalb eines an ein universelles Handstück ankoppelbaren Handstückschafts drehbar gelagert sein muss. Zu diesem Zweck ist vorzugsweise ein Radiallager 50 beispielsweise ein Kugel-, Walz- oder Nadellager mit Innen- und Außenring vorgesehen, das im längsgeschlitzten Drehmoment-Übertragungsbereich 24a auf die Mitnehmerwelle 22 aufgezogen ist und somit gleichzeitig einem Aufspreizen der schnabelförmigen Axialvorsprünge 28 bei einer Drehmomentübertragung auf die Angriffsebenen 8 des Werkzeugschafts 4 entgegenwirkt. Zudem verbessert ein solches Kugellager 50 im längsgeschlitzten Drehmoment-Übertragungsbereich 24a das Einspann-/Ausspannverhältnis am Werkzeugschaft 4, wie dies insbesondere in den Fig. 7a und 7b gezeigt wird.

In der Fig. 7a ist die Einbausituation eines Werkzeugs 1 in einer erfindungsgemäßen Werkzeugaufnahme 20 mit Radiallager 50 im Drehmoment-Übertragungsbereich 24a der Mitnehmerwelle 22 gezeigt. Wie hieraus zu entnehmen ist, wird der distal aus dem Handstückschaft 70 herausragende Werkzeugschaft 4 an wenigstens einem distalen Lager (vorzugsweise zwei distale Lager) 72 und an wenigstens einem proximalen Lager 50, 74 abgestützt, um so Werkzeugaufstandskräfte und Schnittkräfte aufzunehmen, die als Biegekräfte auf den Werkzeugschaft 4 wirken. Wird demnach das wenigstens eine proximale Radiallager 50 in den Drehmoment-Übertragungsbereich 24a der Werkzeugaufnahme 22 gelegt, ergibt sich eine Einspannlänge zwischen distalem und proximalem Lager 72, 50 die deutlich größer ist als die Ausspannlänge zwischen distalem Lager 72 und Werkzeugeingriffssegment 2.

In der Fig. 7b ist hingegen ein Bezugsbeispiel gezeigt, dann, wenn als proximal letztes Radiallager das Lager 74 distal zum Drehmoment-Übertragungsbereich 24a angenommen wird. In diesem Fall verkürzt sich die Einspannlänge gegenüber der Ausspannlänge. Es ist offensichtlich, dass im letzteren Fall gemäß der Fig. 7b die Belastung auf die Radiallager 72, 74 vergrößert sind und daher schneller verschleißen. Auch ist die maximal zulässige Belastung kleiner.

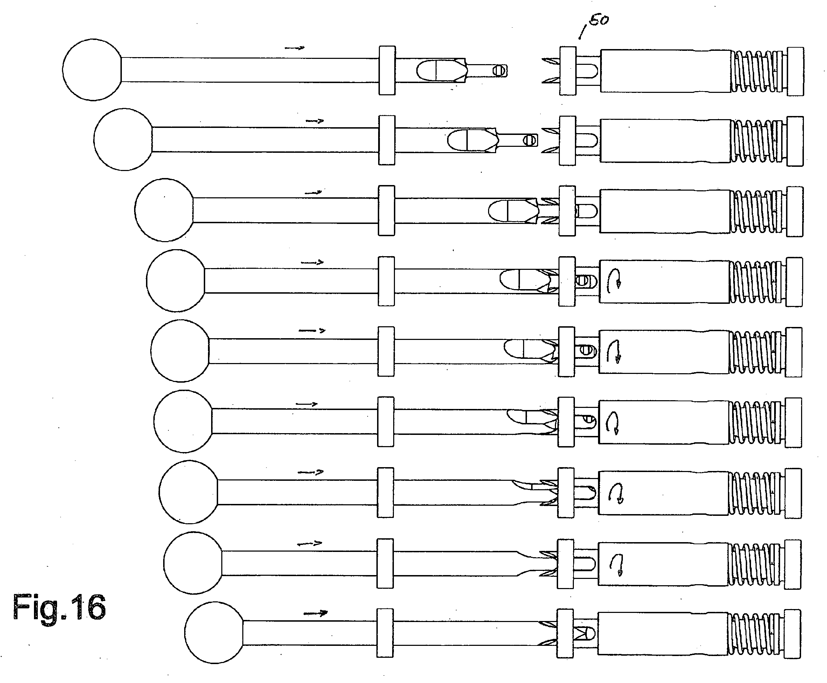

Der Einsetzvorgang des erfindungsgemäßen Werkzeugs 1 in die erfindungsgemäße Werkzeugaufnahme 20 ist im Einzelnen in der Fig. 16 dargestellt.

Zunächst wird der Werkzeugschaft 4 an den Drehmoment-Übertragungsbereich 24a der Werkzeugaufnahme 20 ggf. in einer inkorrekten Relativ-Drehposition herangeführt, wobei in diesem Fall die werkzeugseitigen Abgleitflächen 10 mit den beiden schnabelförmigen Axialvorsprüngen 28 der Werkzeugaufnahme 20 zuerst in Kontakt kommen. Infolge deren Ausrichtung wird die Mitnehmerwelle 22 automatisch gedreht, solange, bis die beiden Angriffsebenen 8 zu den radial äußeren Axialvorsprüngen 28 hinweisen. Jetzt kann der Werkzeugschaft 4 weiter in die Werkzeugaufnahme 20 eingeführt werden, wobei die werkzeugseitigen Angriffsflächen/-ebenen 8 gleitend zwischen den schnabelförmigen Axialvorsprüngen 8 geführt werden. Das in der Fig. 16 ebenfalls gezeigte Radiallager 50 verhindert dabei ein radiales Aufspreizen der schnabel/gabelförmigen Axialvorsprünge/Laschen 28.

Für ein Einsetzen des Werkzeugschafts 4 befindet sich die Schließhülse 46 zunächst in ihrer zurückgezogenen Freigabeposition, in der die Verriegelungskugeln 36 radial nach Außen gedrückt werden können. Letzteres bewerkstelligt das Nachfahrelement 30 (Bolzen), das mit seinem distalen Abschnitt 30a radial zwischen die Verriegelungskugeln 36 durch die Nachfahrfeder 32 gedrückt wird und diese daher radial nach außen hält. Die radial nach außen gedrückten Kugeln 36 halt dann auch die Schließhülse 46 axial in ihrer Freigabeposition.

Beim Eindringen des Werkzeugschafts 4 in die Werkzeugaufnahme 20 stößt jedoch der werkzeugschaftseitige Verriegelungsabschnitt 6b stirnseitig an das Nachfahrelement 30 an und verdrängt dieses in Axialrichtung gegen die Vorspannkraft der Nachfahrfeder 32, solange, bis sich die Taschen/Umfangsnut 12/12a im Verriegelungsabschnitt 6b des Werkzeugschafts 4 im Bereich der Verriegelungskugeln 36 befinden. In diesem Moment werden die Kugeln 36 durch die Schließhülse 46 infolge der axial wirkenden Federvorspannung und einer entsprechenden konischen Formgebung an der Innenumfangsseite der Schließhülse 46 (nicht weiter dargestellt) nach innen gedrückt und kommen somit in der Umfangsnut 12a bzw. den Taschen 12 des Werkzeugschaft 4 zu liegen. Gleichzeitig bewegt sich die Schließhülse 46 durch die Federvorspannung weiter in Richtung nach distal in ihre Verriegelungsposition. Damit ist das Werkzeug 1 axial gesichert und es kann ein Drehmoment von der Torsionswelle 60 über die Mitnahmeelemente 40 und die Mitnehmerwelle 22 auf die Angriffsebenen 8 des Werkzeugschafts 4 übertragen werden.

Um das Werkzeug 1 zu entnehmen, wird die Schließhülse 46 (manuell) gegen die Schließfeder 44 in die Freigabeposition nach proximal zurück gezogen, um die Verriegelungskugeln 36 radial freizugeben. Wird dann der Werkzeugschaft 4 aus der Aufnahme 20 herausgezogen folgt das Nachfahrelement 30 selbsttätig dem Werkzeugschaft 4 infolge der Nachfahrfeder 32 und gelangt so radial zwischen die Verriegelungskugeln 36, um diese radial nach Außen gedrückt zu halten. Die Werkzeugaufnahme 20 verharrt somit in dieser Freigabeposition, um automatisch einen neu eingeführten Werkzeugschaft 4 axial zu verriegeln. Die vorliegende erfindungsgemäße Werkzeugaufnahme 20 kann daher auch als halbautomatische Werkzeugaufnahme (automatisches Verriegeln und manuelles Freigeben) bezeichnet werden.

## Kupplung zwischen Werkzeugaufnahme bzw. Torsionswelle und handstückseitigem Getriebezug

Wie bereits eingangs erläutert wurde, besteht ein Aspekt der vorliegenden Erfindung darin, für unterschiedliche Handstückschäfte immer das gleiche Werkzeug verwenden zu können. Die Handstückschäfte sind dabei so konstruiert, dass sie an ein einziges, universelles Handstück ankuppelbar sind, in welchem der Werkzeugantrieb und/oder der Drehmoment-Übertragungszug/Getriebe untergebracht ist. Dies bedeutet, dass innerhalb des jeweiligen Handstückschafts eine Torsionswelle vormontiert sein muss, an deren distalem Ende die Werkzeugaufnahme vorzugsweise gemäß der vorstehenden Beschreibung und an deren proximalem Ende eine Kupplung vorgesehen sein muss, die zusammen mit dem festen Ankuppeln des Handstückschafts an das Handstück (vorzugsweise an dessen Gehäuse) gleichzeitig mit dem Drehmoment-Übertragungszug in Wirkeingriff kommt, um eine Drehmomentübertragung auf die Torsionswelle zu ermöglichen.

Eine solche Kupplung muss in erster Linie Drehmomente übertragen, aber auch eine axiale Verschiebung der Antriebswelle ermöglichen, damit beispielsweise die Werkzeugaufnahme entriegelt/demontiert werden kann. Außerdem sollte die Kupplung ausreichende Führungseigenschaften besitzen, um zumindest in diesem Bereich weitere Radiallager (Kugellager) zur Abstützung der Kupplung einsparen zu können.

Bisher wurde die betreffende Kupplung zwischen Torsionswelle (innerhalb des auswechselbaren Handstückschafts) und Drehmomentzug (innerhalb des Handstücks) durch einen sogenannten Zweiflach vergleichbar zum vorstehend beschriebenen erfindungsgemäßen Drehmoment-Übertragungsabschnitt zwischen Werkzeugschaft und Werkzeugaufnahme gebildet. Eine solche Kupplung (ohne umgebendes Radiallager) hat jedoch das grundsätzlich Problem einer zu geringen Torsionssteifigkeit im vorgegebenen (engen) Bauraum, was schnell zu einem Versagen der zusammenwirkenden Kupplungsbauteile führen kann. Außerdem sind mit dieser Konstruktion nur unzureichende Führungseigenschaften erreichbar.

Eine Alternative zu dem genannten Zweiflach bildet die allgemein bekannte Kreuzlösung. In diesem Fall ist der männliche Kupplungsteil auf Seiten der Torsionswelle mit achszentral sich kreuzenden Stegen versehen, die in ein entsprechend geformtes weibliches Kupplungsteil einführbar sind, wodurch die für die Drehmomentübertragung nutzbare Fläche an den Seitenflanken jedes Stegs insgesamt vergrößert wird. Jedoch erweist sich diese Lösung ebenfalls als problematisch, in sofern, als dass hierdurch keine optimale Verteilung der Torsionssteifigkeit zwischen dem männlichen und weiblichen Kupplungsteil vorhanden ist, sodass auch hier das maximal übertragbare Drehmoment begrenzt ist.

Um dieses Problem zu lösen, ist demnach eine Querschnittsgeometrie für die Kupplungsteile (männlich und weiblich) erforderlich, welche den gegebenen Bauraum im Fall eines chirurgischen Handstücks der einschlägigen Gattung optimal bezüglich Torsionsträgheitsmoment ausnutzt, gleichzeitig eine axiale Verschiebung der beiden Kupplungsbauteile relativ zueinander ermöglicht und durch die besondere Form nicht in eine Selbsthemmung gerät.

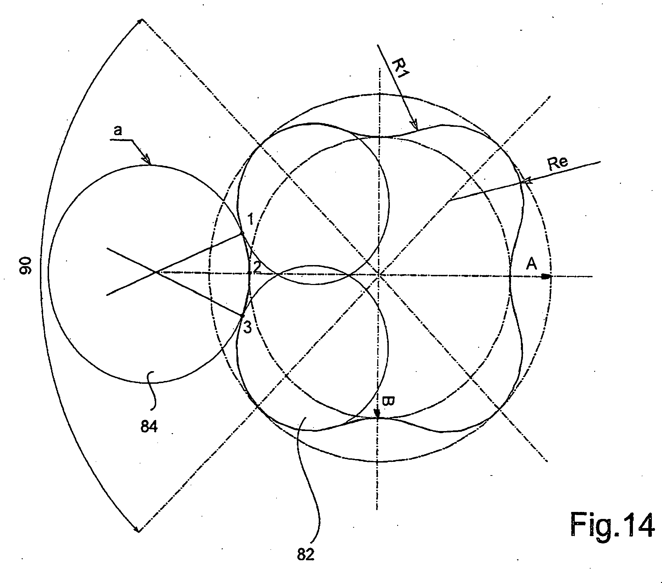

Bei der Entwicklung der erfindungsgemäßen Kupplung hat sich dabei ergeben, dass je kreisähnlicher die Mitnehmerkontur wird, desto weniger Formschluss ist für eine Drehmomentübertragung vorhanden. Aber auch je weniger Ecken die Mitnehmerkontur hat, desto formschlüssiger verbleibt die Kupplungspaarung (bei abnehmendem Widerstandsmoment). Insgesamt erweist sich daher bei einem chirurgischen Instrument dieser Gattung eine Kleeblattkupplung 80 mit vier Blättern als besonders vorteilhafte Kupplungsquerschnittsform. In der Fig. 14 ist ein optimierter Querschnitt einer vierblättrigen Kleeblattform gemäß einem bevorzugten Ausführungsbeispiel der Erfindung dargestellt.

Demzufolge wird die erfindungsgemäße Querschnittsform der Kupplung 80 durch vier gleiche Kreise 82 mit kleinerem Radius Re aufgebaut, welche die vier Ecken der Kupplungsform definieren und die jeweils um 90° winkelversetzt zueinander angeordnet sind. Der Abstand der jeweils benachbarten innerhalb der Kupplungsform liegenden Kreismittelpunkte ist geringfügig kleiner als der einheitliche Kreisdurchmesser Re, sodass sich die jeweils benachbarten Kreise 82 schneiden.

Auf einer Mittelachse zwischen zwei benachbarten, sich schneidenden Kreisen 82 ist außerhalb der Kupplungsform jeweils ein weiterer Kreismittelpunkt gesetzt, wobei um diesen ein Kreis 84 mit größerem Radius Ri gezogen ist. Die jeweilige Position dieses äußeren, weiteren Kreismittelpunkts sowie der größere Radius Ri sind so gewählt, dass die Kontur des äußeren Kreises 84 kontinuierlich in die Kontur der beiden eckseitigen inneren Kreise 82 übergeht und somit unter Ausbildung einer Kavität beide jeweils benachbarten Eckkreise 82 miteinander verbindet. D.h. der äußere Kreis 84 verläuft in den Kontaktpunkten zu den beiden inneren Eckkreisen 82 jeweils tangential, wodurch eine stetige (ecken-/kantenlose) Querschnittskontur mit vier ausgeprägt konvexen Eckkreisen 82 und vier sanft konkaven Seitenkreisen 84 entsteht.

Geometrisch lässt sich die Querschnittskontur gemäß dem bevorzugten Ausführungsbeispiel wie folgt definieren:

Gemäß der Fig. 14 bedeutet der Wert A den Durchmesser eines Umfangskreises mit querschnittsform-zentrischen Kreismittelpunkt und radial äußeren Berührpunkten an allen Eckkreisen. Der Wert B bedeutet das lichte Maß zwischen zwei sich gegenüberliegenden Seitenkreisen, d.h. der Diagonalabstand der querschnittsforminnersten Punkte zweier gegenüberliegender Seitenkreise.

Demnach stehen diese Werte A, B in einem Verhältnis zueinander gemäß der Formel (1):

$$(1) \quad B = kB * A \qquad \text{mit} \qquad 0{,}6 < kB < 0{,}9$$

Der Radius Re jedes Eckkreises steht in einem Verhältnis zum Wert A gemäß der Formel (2):

$$(2) \quad Re = kRe * A \qquad \text{mit} \qquad 0{,}6 < kRe < 0{,}9$$

Der Radius Ri jedes Seitenkreises steht in einem Verhältnis zum Wert A gemäß der Formel (3):

$$(3) \quad Ri = kRi * A \qquad \text{mit} \qquad 0{,}8 < kRi < 1{,}5$$

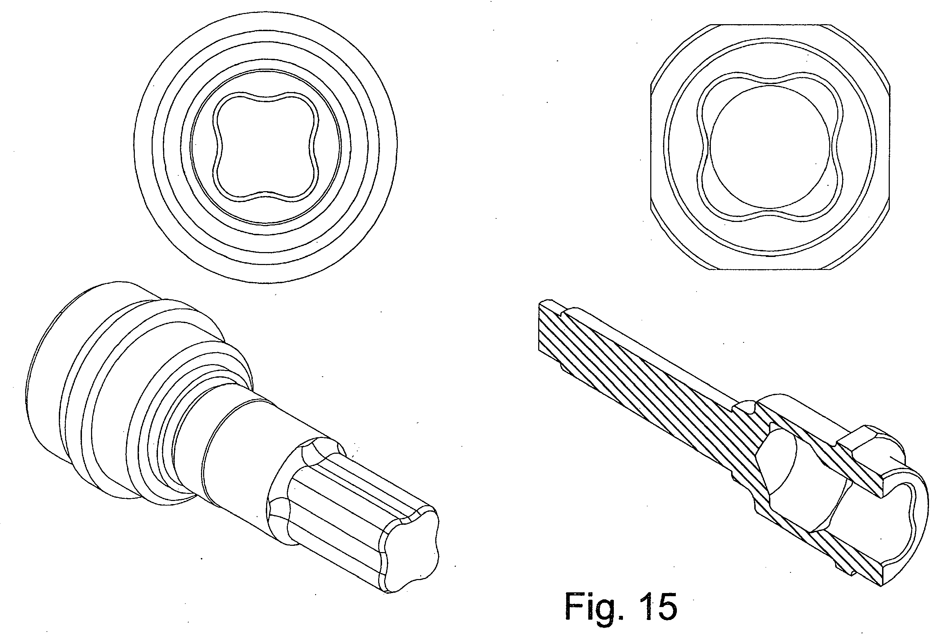

Wie die Fig. 15 zeigt, weisen sowohl der männliche wie auch der weibliche Kupplungsteil eine entsprechende Querschnittsform auf, mit einem bestimmten Übermaß des weiblichen Kupplungsteils.

**Patentansprüche**

**1.** Chirurgisches, Drehmoment übertragendes Instrument mit einem Handstück, an dem ein Handstückschaft (70) angeflanscht oder anflanschbar ist, wobei im Handstück oder im Handstückschaft (70) eine Werkzeugaufnahme (20) für ein wahlweises Aufnehmen eines drehbar im Handstückschaft (70) gelagerten oder lagerbaren chirurgischen Werkzeugs (1) vorgesehen ist zur axialen Sicherung des Werkzeugs (1) in der Werkzeugaufnahme (20) sowie zur Übertragung eines Drehmoments auf das Werkzeug (1), wobei die Werkzeugaufnahme (20) die folgenden Bauteile hat

- eine zumindest abschnittsweise hülsenförmige Mitnehmerwelle (22) mit einem proximalen Einsteckbereich (24d) für eine Torsionswelle (60) sowie distalen Drehmomentübertragungs- und Axialverriegelungsbereichen (24a, 24b) für das Werkzeug (1) und
- eine die Mitnehmerwelle (22) verschiebbar sowie vorzugsweise drehbar umgebende Schließhülse (46) mit einem distalen Sperrabschnitt, der für ein axiales Verriegeln und Freigeben des Werkzeugs (1) auf den Axialverriegelungsbereich der Mitnehmerwelle (24b) einwirkt und **gekennzeichnet durch** einen proximalen Arretierabschnitt, der für ein Drehmoment sowie Axialkräfte übertragendes Arretieren und Lösen der Werkzeugaufnahme (20) an und von der Torsionswelle (60) auf den Einsteckbereich (24d) der Mitnehmerwelle (22) einwirkt.

2. Chirurgisches, Drehmoment übertragendes Instrument nach Anspruch 1, **gekennzeichnet durch** zumindest ein, vorzugsweise mehrere in Umfangsrichtung beabstandete Mitnahmeelemente (40), die im Einsteckbereich (24d) zwischen die Mitnehmerwelle (22) und die Torsionswelle (60) formschlüssig eingefügt sind und **durch** die Schließhülse (46) in einer axialen Verriegelungsposition radial nach Außen gesichert oder sicherbar sind.

3. Chirurgisches, Drehmoment übertragendes Instrument nach Anspruch 2, **dadurch gekennzeichnet, dass** die Mitnahmeelemente (40) vorzugsweise in Form von Kugeln, oder stirnseitig abgerundeten Walzen, Zylindern oder Tonnen in Aufnahmetaschen (64) am Außenumfang der Torsionswelle (60) sowie in radialen Durchbrüchen oder Öffnungen (38) im Einsteckbereich (24d) der Schließhülse (46) sitzen, um von der Torsionswelle (60) zumindest Drehmomente unmittelbar auf die Mitnehmerwelle (22) zu übertragen.

4. Chirurgisches, Drehmoment übertragendes Instrument nach Anspruch 3, **dadurch gekennzeichnet, dass** die Schließhülse (46) in ihrem proximalen Arretierabschnitt zumindest eine und vorzugsweise eine Anzahl von umfangsseitigen Befüllöffnungen (48) für das Einsetzen der Mitnahmeelemente (40) in ihrer einen axialen Freigabe- sowie rotatorischen Befüllposition hat.

5. Chirurgisches, Drehmoment übertragendes Instrument nach einem der vorstehenden Ansprüche, **dadurch gekennzeichnet, dass** der distale Drehmoment-Übertragungsbereich (24a) durch einen axialen Längsschlitz in die in diesem Abschnitt hülsenförmige Mitnehmerwelle (22) gebildet ist, wodurch zwei axial sich erstreckende, einen Werkzeugaufnahmespalt definierende Vorsprünge oder Zungen (28) entstehen, die für eine Formschlussverbindung mit einem eingesetzten Werkzeug vorgesehen sind.

6. Chirurgisches, Drehmoment übertragendes Instrument nach Anspruch 5, **dadurch gekennzeichnet, dass** im proximalen Anschluss an den Drehmoment-Übertragungsbereich (24a) der Axialverriegelungsbereich (24b) angeordnet ist, unter anderem bestehend aus zumindest einer, vorzugsweise einer Anzahl winkelbeabstandeter Radial-Durchgangsbohrungen (34), in die Verriegelungselemente vorzugsweise in Form von Kugeln (36) eingesetzt sind, welche durch die Schließhülse (46) in ihrer axialen Verriegelungsposition radial nach innen gedrückt werden.

7. Chirurgisches, Drehmoment übertragendes Instrument nach Anspruch 6, **dadurch gekennzeichnet, dass** die Schließhülse (46) an ihrem distalen Endabschnitt eine innere radiale Aufweitung (46a) hat zum radialen Freigeben der Kugeln in ihrer axialen Freigabeposition und zum Befüllen der Radial-Durchgangsbohrungen (34) mit den Verriegelungskugeln (36) in ihrer axialen Freigabe- sowie rotatorischen Befüllposition.

8. Chirurgisches, Drehmoment übertragendes Instrument nach einem der Ansprüche 5 bis 7, **gekennzeichnet durch** eine in der Mitnehmerwelle (22) ausgeformte Aufnahmekammer (24c) proximal zum Verriegelungsbereich (24b) für ein Nachfahrelement (30), das einen axialen, vorzugsweise bolzenförmigen Eingriffsabschnitt (30a) hat, der **durch** eine axial wirkende Nachfahrfeder (32) in Richtung radial zwischen die vorzugsweise kugelförmigen Verriegelungselemente (36) gedrängt wird.

9. Chirurgisches, Drehmoment übertragendes Instrument nach Anspruch 8, **dadurch gekennzeichnet, dass** das Nachfahrelement (30) dafür vorgesehen ist, bei nicht eingesetztem Werkzeug die Verriegelungselemente (36) radial nach Außen zu drücken und dabei die Schließhülse (46) in ihrer axialen Freigabeposition zu halten und durch Einsetzten eines Werkzeugs (1) axial gegen die Nachfahrfeder (32) verschoben zu werden, um ein radiales Einrücken der Verriegelungselemente (36) für ein Axialverriegeln des Werkzeugs (1) sowie ein axiales Verschieben der Schließhülse (46) in ihre Verriegelungsposition zu erlauben, in welcher die Verriegelungselemente (36) radial einwärts gegen das Werkzeug (1) gedrückt bleiben.

10. Chirurgisches, Drehmoment übertragendes Instrument nach einem der vorstehenden Ansprüche, **dadurch gekennzeichnet, dass** ein von der Torsionswelle (60) eingebrachtes Drehmoment über eine Anzahl von umfangsseitig an der Torsionswelle (60) angeordnete Mitnahmeelementen (40) auf die Mitnehmerwelle (22) und von dieser unmittelbar auf ein darin aufgenommenes Werkzeug (1) übertragen wird, wobei die Axialsicherung des Werkzeugs (1) in der Mitnehmerwelle (22) außerhalb dieses Drehmoment-Übertragungszugs durch axial wirkende Verriegelungselemente (36) zwischen der Mitnehmerwelle (22) und dem Werkzeug (1) erfolgt.

## Claims

1. A surgical torque-transferring instrument comprising a handpiece to which a handpiece shaft (70) is flanged or can be flanged, a tool mounting (20) being provided, in the handpiece or in the handpiece shaft (70), for selectively receiving a surgical tool (1) which is rotatably supported in the handpiece shaft (70) or can be rotatably supported therein, for axially securing the tool (1) in the tool mounting (20) as well as for transferring a torque to the tool (1), wherein the tool mounting (20) comprises the following components:

   - an entrainment shaft (22) which is formed like a sleeve at least in sections and comprises a proximal plug-in zone (24d) for a torsion rod (60) as well as distal torque transmission and axial locking zones (24a, 24b) for the tool (1) and
   - a closure sleeve (46) surrounding the entrainment shaft (22) in a shiftable manner and preferably in a rotatable fashion, said closure sleeve comprising a distal blocking section which acts on the axial locking zone of the entrainment shaft (24b) for axially locking and releasing the tool (1), and **characterized by** a proximal arresting section acting on the plug-in zone (24d) of the entrainment shaft (22) for arresting the tool mounting (20) on the torsion rod (60) to transfer torques and axial forces and for releasing the tool mounting (20) from the torsion rod (60).

2. The surgical torque-transferring instrument according to claim 1, **characterized by** at least one entrainment element (40), preferably several entrainment elements (40) spaced in circumferential direction, which is/are inserted in the plug-in zone (24d) in a form-fitting manner between the entrainment shaft (22) and the torsion rod (60) and are radially secured towards outside by the closure sleeve (46) in an axial locking position or can be secured in such manner.

3. The surgical torque-transferring instrument according to claim 2, **characterized in that** the entrainment elements (40), preferably realized in the form of balls, or rollers, cylinders or barrels rounded on the end face, are located in accommodation pockets (64) on the outer circumference of the torsion rod (60) as well as in radial apertures or openings (38) in the plug-in zone (24d) of the closure sleeve (46), in order to directly transfer at least torques from the torsion rod (60) to the entrainment shaft (22).

4. The surgical torque-transferring instrument according to claim 3, **characterized in that** the closure sleeve (46) has provided its proximal arresting section with at least one fill opening (48) and preferably a number of circumferential fill openings (48) for inserting the entrainment elements (40) in its one axial release position and rotary filling position.

5. The surgical torque-transferring instrument according to any of the preceding claims, **characterized in that** the distal torque-transferring zone (24a) is formed by an axial longitudinal slit in the entrainment shaft (22) which is formed like a sleeve in said portion, producing two axially extending protrusions or tongues (28) which define a tool mounting gap and are provided for a form-fitting connection with an inserted tool.

6. The surgical torque-transferring instrument according to claim 5, **characterized in that** the axial locking zone (24b) is arranged in the proximal extension of the torque-transferring zone (24a), said axial locking zone consisting inter alia of at least one radial through-hole, preferably a number of angularly spaced radial through-holes (34) receiving locking elements preferably in the form of balls (36) which are pushed radially inwards by the closure sleeve (46) in its axial locking position.

**7.** The surgical torque-transferring instrument according to claim 6, **characterized in that** the closure sleeve (46) has its distal end portion provided with an inner radial widening (46a) for radially releasing the balls in its axial release position and for filling the radial through-holes (34) with the locking balls (36) in its axial release position and rotary filling position.

**8.** The surgical torque-transferring instrument according to any of the claims 5 to 7, **characterized by** an accommodation chamber (24c) which is formed in the entrainment shaft (22), is disposed proximal relative to the locking zone (24b) and holds a follower element (30) which comprises an axial, preferably bolt-shaped engagement portion (30a) being urged in the axial direction between the preferably ball-shaped locking elements (36) by an axially acting follower spring (32).

**9.** The surgical torque-transferring instrument according to claim 8, **characterized in that** the follower element (30) is provided for urging the locking elements (36) radially outward if the tool is not inserted and in doing so retaining the closure sleeve (46) in its axial release position, and for being axially shifted against the follower spring (32) by the insertion of a tool (1) in order to allow a radial inward motion of the locking elements (36) for axially locking the tool (1) as well as an axial displacement of the closure sleeve (46) into its locking position in which the locking elements (36) continue to be pushed radially inward against the tool (1).

**10.** The surgical torque-transferring instrument according to any of the preceding claims, **characterized in that** a torque introduced by the torsion rod (60) is transferred to the entrainment shaft (22) through a number of entrainment elements (40) which are circumferentially arranged on the torsion rod (60), and from said entrainment shaft directly to a tool (1) received therein, the axial securement of the tool (1) in the entrainment shaft (22) being effected outside of said torque transmission train by axially acting locking elements (36) between the entrainment shaft (22) and the tool (1).

## Revendications

**1.** Instrument chirurgical à transmission de couple de rotation, comprenant une pièce à main, au niveau de laquelle une tige de pièce à main (70) est attachée ou peut être attachée par une bride, dans lequel est prévu, dans la pièce à main ou dans la tige de pièce à main (70), un logement d'outils (20) pour loger au choix un outil (1) chirurgical monté ou pouvant être monté de manière à pouvoir tourner dans la tige de pièce à main (70) afin d'immobiliser de manière axiale l'outil (1) dans le logement d'outils (20) et afin de transmettre également un couple de rotation sur l'outil (1), dans lequel le logement d'outils (20) a les composants suivants

- un arbre entraîneur (22) présentant au moins par endroits une forme de manchon, pourvu d'une zone d'emboîtement (24d) proximale pour un arbre de torsion (60) ainsi que de zones de transmission de couple de rotation et de verrouillage axial (24a, 24b) distales pour l'outil (1), et
- un manchon de fermeture (46) entourant de manière à pouvoir être coulissé et de préférence de manière à pouvoir tourner également l'arbre entraîneur (22), pourvu d'une section de blocage distale, qui agit sur la zone de verrouillage axial de l'arbre entraîneur (24b) en vue d'un verrouillage axial et d'un déblocage de l'outil (1), et **caractérisé par** une section d'arrêt proximale, qui agit sur la zone d'emboîtement (24d) de l'arbre entraîneur (22) en vue d'un couple de rotation ainsi qu'en vue d'un arrêt transmettant des forces axiales et d'un détachement du logement d'outils (20) au niveau et de l'arbre de torsion (60).

**2.** Instrument chirurgical à transmission de couple de rotation selon la revendication 1, **caractérisé par** au moins un, de préférence plusieurs éléments d'entraînement (40) espacés dans la direction périphérique, qui sont insérés par complémentarité de forme dans la zone d'emboîtement (24d) entre l'arbre entraîneur (22) et l'arbre de torsion (60) et qui sont immobilisés ou peuvent être immobilisés de manière radiale vers l'extérieur par le manchon de fermeture (46) dans une position de verrouillage axiale.

**3.** Instrument chirurgical à transmission de couple selon la revendication 2, **caractérisé en ce que** les éléments d'entraînement (40) reposent de préférence sous la forme de billes, ou de rouleaux arrondis côté frontal, de cylindres ou de tonnelets dans des compartiments de logement (64) au niveau de la périphérique extérieure de l'arbre de torsion (60) ainsi que dans des ajours ou des orifices (38) radiaux dans la zone d'emboîtement (24d) du manchon de fermeture (46) afin de transmettre depuis l'arbre de torsion (60) au moins des couples de rotation directement sur l'arbre entraîneur (22).

**4.** Instrument chirurgical à transmission de couple selon la revendication 3, **caractérisé en ce que** le manchon de fermeture (46) a, dans sa section d'arrêt proximale, au moins un et de préférence un nombre donné d'orifices de remplissage (48) côté périphérie pour l'insertion des éléments d'entraînement (40) dans leur position de déblocage axiale ainsi que dans leur position de remplissage de rotation.

**5.** Instrument chirurgical à transmission de couple selon l'une quelconque des revendications précédentes, **caractérisé en ce que** la zone de transmission de couple de rotation (24a) distale est formée par une entaille longitudinale axiale dans l'arbre entraîneur (22) présentant une forme de manchon dans ladite section, ce qui permet de former deux parties faisant saillie ou languettes (28) s'étendant de manière axiale, définissant une fente de logement d'outils, lesquelles sont prévues en vue d'un assemblage par complémentarité de forme à un outil inséré.

**6.** Instrument chirurgical à transmission de couple selon la revendication 5, **caractérisé en ce qu'**est disposée dans le prolongement proximal de la zone de transmission de couple de rotation (24a), la zone de verrouillage axial (24b), constituée entre autres d'au moins un, de préférence d'un nombre donné d'alésages de passage radial (34) espacés selon un angle donné, dans lesquels des éléments de verrouillage sont insérés de préférence sous la forme de billes (36), qui sont poussés de manière radiale vers l'intérieur par le manchon de fermeture (46) dans leur position de verrouillage axiale.

**7.** Instrument chirurgical à transmission de couple selon la revendication 6, **caractérisé en ce que** le manchon de fermeture (46) a, au niveau de sa section d'extrémité distale, un élargissement (46a) radial intérieur pour débloquer de manière radiale les billes dans leur position de déblocage axiale et pour remplir les alésages de passage (34) radiaux des billes de verrouillage (36) dans leur position de déblocage axiale ainsi que dans leur position de remplissage de rotation.

**8.** Instrument chirurgical à transmission de couple selon l'une quelconque des revendications 5 à 7, **caractérisé par** une chambre de logement (24c) formée dans l'arbre entraîneur (22) de manière proximale par rapport à la zone de verrouillage (24b) pour un élément de déplacement ultérieur (30), qui a une section de prise (30a) axiale, de préférence présentant une forme de boulon, laquelle est refoulé par un ressort de déplacement ultérieur (32) agissant de manière axiale dans une direction de manière radiale entre les éléments de verrouillage (36) présentant de préférence une forme de bille.

**9.** Instrument chirurgical à transmission de couple selon la revendication 8, caractérisé e ce que l'élément d'ajustement ultérieur (30) est prévu pour pousser, lorsque l'outil n'est pas inséré, les éléments de verrouillage (36) de manière radiale vers l'extérieur et pour ainsi maintenir le manchon de fermeture (46) dans sa position de déblocage axiale et pour les faires coulisser de manière axiale contre le ressort d'ajustement ultérieur (32) par l'insertion d'un outil (1) afin de permettre un embrayage radial des éléments de verrouillage (36) pour un verrouillage axial de l'outil (1) ainsi qu'un coulissement axial du manchon de fermeture (46) dans sa position de verrouillage, dans laquelle les éléments de verrouillage (36) restent radialement à l'intérieur en étant poussés contre l'outil (1).

**10.** Instrument chirurgical à transmission de couple selon l'une quelconque des revendications précédentes, **caractérisé en ce qu'**un couple de rotation pratiqué par l'arbre de torsion (60) est transmis par l'intermédiaire d'un nombre donné d'éléments d'entraînement (40) disposés côté périphérique sur l'arbre de torsion (60) sur l'arbre entraîneur (22) et par ce dernier directement sur un outil (1) logé dans ce dernier, l'immobilisation axiale de l'outil (1) dans l'arbre entraîneur (22) étant effectuée à l'extérieur de ladite chaîne de transmission de couple de rotation par des éléments de verrouillage (36) agissant de manière axiale entre l'arbre entraîneur (22) et l'outil (1).

FIG. 1

Fig.3

Fig.4a

Fig.2

Fig.4b

A-A

Fig.5

Fig.6

Fig.7a

Fig.7b

8

12a

6c

6a

10

6b

Fig.8

EP 2 892 441 B1

EP 2 892 441 B1

6a    6c 6b 12

D
(klein)                    d
                    kurz

6a    6c    6b    12

D
(größer)                    d
                    länger

Fig.9

Fig.10

Fig.11

Fig.12

Fig.13

Fig.14

Fig. 15

Fig.16

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10311455 A1
- US 2011098688 A1